# EUROPÄISCHE PATENTSCHRIFT

(11) **EP 1 860 158 B1**
(45) Veröffentlichungstag und Bekanntmachung des Hinweises auf die Patenterteilung: **04.12.2013**
(21) Anmeldenummer: 07010046.6
(22) Anmeldetag: 21.05.2007
(51) Int. Cl.: C09C 1/00

(54) **Partikel umfassend Substrate und eine anionbindende Schicht**
Particles comprising a substrate and an anion-binding layer
Particule comprenant un substrat et une couche liant des anions

(30) Priorität: 24.05.2006 DE 102006024289
(43) Veröffentlichungstag der Anmeldung: 28.11.2007
(73) Patentinhaber: Merck Patent GmbH, 64293 Darmstadt (DE)
(72) Erfinder: Handrosch, Carsten, Dr., 64367 Muehltal (DE); Rudolph, Thomas, Dr., 64291 Darmstadt (DE); Buchholz, Herwig, Dr., 60599 Frankfurt/Main (DE); Anzali, Soheila, Dr., 64342 Seeheim-Jugenheim (DE)

(56) Entgegenhaltungen:
- EP-A1- 1 481 658
- EP-A2- 1 847 571

## Beschreibung

Die vorliegende Erfindung betrifft Partikel umfassend Substrate, und eine Anion-bindende Schicht, wobei die Anion-bindende Schicht einen oder mehrere Anion-bildende organische Wirkstoffe enthält. Weiterhin betrifft die vorliegende Erfindung Verfahren zur Herstellung von Partikeln sowie deren Verwendung in Kosmetika, Pharmazeutika, Formulierungen, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

Wirkstoffe spielen in der Kosmetik und bei pharmazeutischen Produkten eine herausragende Rolle. Beispiele für kosmetische Wirkstoffe sind UV-Filter, entzündungshemmende Substanzen etc. In der Regel handelt es sich bei den genannten Wirkstoffen um organische funktionelle Wirkstoffe. Diese organischen funktionellen Wirkstoffe werden üblicherweise in Form einer Formulierung appliziert oder verabreicht. Beispiele solcher Formulierungen sind in der Kosmetik beispielsweise Cremes, Salben oder Gele, im Falle von pharmazeutischen Wirkstoffen beispielsweise Tabletten, Dragees, Lösungen aber auch Salben.

Vielfach sind die genannten Wirkstoffe in den Formulierungen aber nicht stabil genug bzw. können mit anderen Bestandteilen der Formulierung reagieren. Weiterhin ist die Dosierung der Wirkstoffe bei Anwendung der Formulierungen vielfach nicht steuerbar, z.B. kommt die Haut bei Aufbringung einer Salbe mit der gesamten Konzentration des Wirkstoffs in Berührung, auch wenn dies anfänglich nicht gewünscht sein mag. Es gibt eine Reihe von Ansätzen, die Freisetzung oder Stabilisierung von Wirkstoffen zu verbessern bzw. zu regulieren. Dies erfolgt vielfach durch Einschluss der Wirkstoffe in Trägern, die die Wirkstoffe dann gezielt freisetzen sollen.

Es ist daher die Aufgabe der vorliegenden Erfindung, Partikel bereitzustellen, die in der Lage sind Wirkstoffe zu stabilisieren bzw. gezielt freizusetzen. Ferner ist es die Aufgabe der vorliegenden Erfindung, durch geeignete Auswahl von Substrat und Anion-bindender Schicht, synergistische Wirkprofile zu erzielen

Überraschenderweise wird die oben genannte Aufgabe durch Partikel der vorliegenden Erfindung gelöst.

Demgemäß sind Partikel umfassend Substrate und eine Anion-bindende Schicht, die ein oder mehrere Anion-bildende organische Wirkstoffe enthält, ein erster Gegenstand der vorliegenden Erfindung.
Durch die erfindungsgemäßen Partikel können zweierlei, grundsätzlich unterschiedliche Funktionen in Bezug auf den gebundenen anionischen Wirkstoff verwirklicht werden: Bei niedrigem pH-Wert (< 6) löst sich die Fixierungsschicht sukzessive auf, woraus sich eine controlled-release-Funktion der Partikel ergibt. Bei höherem pH-Wert (> 6) ist die Fixierungsschicht hingegen stabil und übt so eine schützende und stabilisierende Funktion auf den gebundenen anionischen Wirkstoff aus. Es handelt sich also bei den Partikeln gemäß der vorliegenden Erfindung insbesondere um multifunktionelle Hybridpartikel.

Aufgrund der vorteilhaften Eigenschaften eignen sich die erfindungsgemäßen Partikel universell für eine große Anzahl unterschiedlichster Anwendungen. Gegenstand der vorliegenden Erfindung ist demgemäß auch die Verwendung dieser Partikel in Kosmetika, Pharmazeutika, Formulierungen, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

Die erfindungsgemäßen Partikel basieren auf Substraten, die grundsätzlich jede Form haben können. Beispiele entsprechend geformter Substrate sind plättchenförmige, sphärische oder nadelförmige Substrate, aber auch unregelmäßig geformte Substrate können eingesetzt werden. Unter sphärisch wird im Sinne der vorliegenden Erfindung kugelförmig aber auch unsymmetrisch kugelförmig, z.B. ellipsenförmig, verstanden.

Geeignete plättchenförmige Substrate sind beispielsweise Glasplättchen, SiO₂-Plättchen, Al₂O₃-Plättchen, BiOCI-Plättchen, Graphitplättchen, künstliches oder natürliches plättchenförmiges Eisenoxid oder plättchenförmige Metalle. Geeignete Metallplättchen können unter anderem aus Aluminium, Titan, Bronze, Stahl oder Silber bestehen, vorzugsweise aus Aluminium und/oder Titan. Die Metallplättchen können dabei durch entsprechende Behandlung passiviert sein.

Die Größe der plättchenförmigen Basissubstrate ist an sich nicht kritisch und kann auf den jeweiligen Anwendungszweck abgestimmt werden. Der Durchmesser der plättchenförmigen Substrate liegt im Mittel üblicherweise zwischen 1 und 500 µm, vorzugsweise zwischen 5 und 200 µm und insbesondere 10 - 200 µm. Bevorzugte kleinere Partikelgrößen sind weiterhin solche im Bereich von 1 - 100 µm, insbesondere 5 - 60 µm und 1 - 15 µm. Ihre mittlere Dicke beträgt zwischen 0,05 und 10 µm und bevorzugt 0,1 bis 1,0 µm. Das mittlere Aspektverhältnis der plättchenförmigen Substrate, das heißt das Verhältnis vom mittleren Längenmesswert, der hier dem mittleren Durchmesser entspricht, zum mittleren Dickenmesswert, beträgt üblicherweise 5 bis 750, bevorzugt 10 bis 300 und besonders bevorzugt 20 bis 200.

Vorzugsweise handelt es sich im Falle von plättchenförmigen Substraten um Glasplättchen, SiO₂-Plättchen oder Al₂O₃-Plättchen und, aufgrund ihrer besonders glatten Oberfläche und ihres sehr hohen Reflexionsvermögens, ganz besonders bevorzugt um Glasplättchen.

Bei den bevorzugt eingesetzten SiO₂-Plättchen handelt es sich um synthetische SiO₂-Plättchen, die über eine einheitliche Schichtdicke verfügen und vorzugsweise gemäß der internationalen Anmeldung WO 93/08237 auf einem endlosen Band durch Verfestigung und Hydrolyse einer Wasserglaslösung hergestellt werden. Unter einheitlicher Schichtdicke wird dabei eine Schichtdickentoleranz von 3 bis 10%, bevorzugt von 3 bis 5 % der Gesamttrockenschichtdicke der Partikel verstanden. Die plättchenförmigen Siliziumdioxidpartikel liegen im Allgemeinen in amorpher Form vor. Synthetische Plättchen dieser Art haben gegenüber natürlichen Materialien, wie z.B. Glimmer, den Vorteil, dass die Schichtdicke im Hinblick auf die gewünschten Effekte eingestellt werden kann und die Schichtdickentoleranz begrenzt ist.

Grundsätzlich können alle, dem Fachmann bekannte, Glas-Typen und - formen (z. B. Plättchen, Kugeln, Fasern etc.) für die erfindungsgemäßen Partikel verwendet werden. Ganz besonders bevorzugt sind Glasplättchen, welche aus allen dem Fachmann bekannten Glastypen bestehen können, insbesondere Ca-Al-Borosilikatgläsem, wie z.B. Fensterglas, C-Glas, E-Glas, ECR-Glas, Duran^{®}-Glas. Laborgeräteglas oder optisches Glas. Insbesondere bevorzugt ist E-Glas oder ECR-Glas, aber auch Eisen, Bismuth, Niob, Zinn und/oder Titan-haltige Gläser. Der Brechungsindex der Plättchen liegt vorzugsweise bei 1,20 - 2,20, insbesondere bei 1,50 - 1,70.

Als plättchenförmige Substrate kommen demnach Substrate auf Basis von Ca-Al-Borosilikat (z.B. RONASTAR® von Firma Merck KGaA), SiO₂ (z.B. COLORSTREAM® von Firma Merck KGaA), Al₂O₃ (z.B. XIRALLIC® von Firma Merck KGaA), natürlichem blättchenförmigem Eisenoxid (z.B. MIOX® von Fa. Kärntner Montan Industrie), künstlichem oder natürlichem Graphit, künstlichem blättchenförmigem Eisenoxid (z.B. TAROX® von Firma Titan Kogyo), oder metallischem Aluminium in Frage.

Sphärische Substrate basieren insbesondere auf Ca-Al-Borosilikat, SiO₂, TiO₂, Al₂O₃, GeO₂, ZrO₂, ZnO, B₂O₃, Ga₂O₃, In₂O₃, SnO₂, aber auch auf (Ca/Mg)₂(OH)(PO₄), Kaolin, BaSO₄, Kreide, MgCO₃, BiOCl (z.B. Biron® ESQ). Ferner sind auch sphärische Farbpigmente wie z. B. natürliches oder künstliches Eisenoxid, Ultramarinblau, Carbon Black, Chromoxidgrün, Kobaltblau, Berliner Blau und Manganviolett als Substrate geeignet. Insbesondere sphärische Substrate aus ZnO oder TiO₂ wirken als UV-Filter. Partikel auf Basis dieser Substrate eignen sich in besonderer Weise, um multifunktionelle Partikel herzustellen. Das Substrat zeigt dabei eine funktionelle Wirkung, die durch die in der Anion-bindenden Schicht gebundenen Wirkstoffe und deren Wirkung ergänzt wird.

Sphärische Kapseln der oben genannten Materialien, die organische und/oder anorganische Verbindungen und Materialien einschließen, sind ebenfalls als sphärische Substrate geeignet. Die eingekapselte Verbindung kann beispielsweise ausgewählt sein aus der Gruppe der UV-Filter. Bevorzugte Kapseln weisen Kapselwände auf, wie sie beispielsweise durch einen Prozess, beschrieben in WO 00/09652, WO 00/72806 und WO 00/71084 erhalten werden. Bevorzugt handelt es sich bei den Kapselwänden um Hüllen auf Basis von SiO₂ oder TiO₂. Ein Beispiel für derartige Kapseln sind die Eusolex^{®}UV-Pearls^{™} (Merck KGaA, Darmstadt).

Partikel auf Basis sphärischer Substrate sind insbesondere für kosmetische und pharmazeutische Anwendungen vorteilhaft. Abhängig vom Material zeigen sphärische Partikel der vorliegenden Erfindung gute Faltenverdeckende Effekte, ein gutes Hautgefühl und können sowohl als Füllstoff als auch als aktives Agens eingesetzt werden. Weiterhin wird der Glanz der Haut verringert und der Haut eine weichere Erscheinung verliehen. Durch den Roll- und Gleiteffekt der sphärischen Partikel wird das Hautgefühl wesentlich verbessert.

Die Größe der sphärischen Basissubstrate ist ebenfalls an sich nicht kritisch und kann auf den jeweiligen Anwendungszweck abgestimmt werden. Der Durchmesser der sphärischen Partikel liegt üblicherweise zwischen 1 nm und 2000 µm, vorzugsweise zwischen 5 nm und 1000 µm und insbesondere bevorzugt zwischen 10 nm und 500 µm. Sphärische Metalloxide, insbesondere Metalloxide mit UV-Filternder Aktivität, haben vorzugsweise einen durchschnittlichen Durchmesser von 5 bis 1000 µm, insbesondere 8 bis 500 nm und ganz besonders bevorzugt von 8 to 300 nm.

Darüber hinaus eignen sich im Rahmen der vorliegenden Erfindung auch nadelförmige Substrate. Beispiele hierfür sind Glasfasern, Kunststofffasern und Pigmente mit nadelförmiger Kristallstruktur, wie z.B. gelbes Eisenoxid (Goethit).

Die oben genannten Substrate können auch als Gemische vorliegen (z.B. binäre oder ternäre Gemische), das heißt z.B. plättchenförmige Substratpartikel liegen in Mischung mit sphärischen und/oder nadelförmigen Substratpartikeln vor. Das Mischungsverhältnis zwischen sphärischen und nicht-sphärischen Teilchen, beispielsweise zwischen plättchen- und nadelförmigen Teilchen ist nicht kritisch und kann der Anwendung angepasst werden, es kann z.B. zwischen 1:99 und 99:1, insbesondere zwischen 10:90 und 90:10 und speziell zwischen 1:9 und 9:1 Gewichtsanteile gewählt werden. Durch solche Gemische lassen sich gezielt bestimmte physikalische Eigenschaften, wie z.B. der Glanzgrad, das Deckvermögen, oder das Hautgefühl des Endproduktes einstellen.

Die oben genannten Substrate können mit Ionen oder Elementen dotiert sein, welche zu einer Färbung des Substrates führen. Darüber hinaus können die Substrate auch mit Ionen oder Elementen dotiert sein, welche, außer/neben der Farbe, eine bestimmte physikalische Eigenschaft des Substrates hervorrufen, z. B. Leitfähigkeit, erhöhter Brechungsindex, Fluoreszenz, Phosphoreszenz, Magnetismus, NLO-Eigenschaften, IR- bzw. UV-Reflexion/Absorption etc. Dabei sind die aufgeführten, durch lonen- oder Elementdotierung induzierten, physikalischen Eigenschaften nur zur Erläuterung der vorliegenden Erfindung gedacht, ohne sie zu begrenzen.

In einer weiteren Ausführungsform der vorliegenden Erfindung können auf den oben genannten Substraten und unterhalb der Anion-bindenden Schicht eine oder mehrere transparente, semitransparente und/oder opake Schichten aus Metalloxiden, Metalloxidhydraten, Metallsuboxiden, Metallen, Metallfluoriden, Metallnitriden, Metalloxynitriden oder Mischungen dieser Materialien aufgebracht sein. Die Metalloxid-, Metalloxidhydrat-, Metallsuboxid-, Metall-, Metallfluorid-, Metallnitrid-, Metalloxynitridschichten oder die Mischungen hieraus können niedrig- (Brechzahl < 1.8) oder hochbrechend (Brechzahl ≥ 1.8) sein. Als Metalloxide und Metalloxidhydrate eignen sich alle dem Fachmann bekannten Metalloxide oder Metalloxidhydrate, wie z. B. Aluminiumoxid, Aluminiumoxidhydrat, Siliziumoxid, Siliziumoxidhydrat, Eisenoxid, Zinnoxid, Ceroxid, Zinkoxid, Zirkoniumoxid, Chromoxid, Titanoxid, insbesondere Titandioxid, Titanoxidhydrat sowie Mischungen hieraus, wie z.B. Ilmenit oder Pseudobrookit. Als Metallsuboxide können beispielsweise die Titansuboxide eingesetzt werden. Als Metalle eignen sich z.B. Chrom, Aluminium, Nickel, Silber, Gold, Titan, Kupfer oder Legierungen, als Metallfluorid eignet sich beispielsweise Magnesiumfluorid. Als Metallnitride oder Metalloxynitride können beispielsweise die Nitride oder Oxynitride der Metalle Titan, Zirkonium und/oder Tantal eingesetzt werden. Bevorzugt werden Metalloxid-, Metall-, Metallfluorid und/oder Metalloxidhydratschichten und ganz besonders bevorzugt Metalloxid- und/oder Metalloxidhydratschichten auf den Träger aufgebracht. Weiterhin können auch Mehrschichtaufbauten aus hoch- und niedrigbrechenden Metalloxid-, Metalloxidhydrat-, Metall- oder Metallfluoridschichten vorliegen, wobei sich vorzugsweise hoch- und niedrigbrechende Schichten abwechseln. Insbesondere bevorzugt sind Schichtpakete aus einer hoch- und einer niedrigbrechenden Schicht, wobei auf dem Träger eines oder mehrere dieser Schichtpakete aufgebracht sein können. Die Reihenfolge der hoch- und niedrigbrechenden Schichten kann dabei an das Substrat angepasst werden, um das Substrat in den Mehrschichtaufbau mit einzubeziehen. In einer weiteren Ausführungsform können die Metalloxid-, Metalloxidhydrat-, Metallsuboxid-, Metall-, Metallfluorid-, Metallnitrid-, Metalloxynitridschichten mit Farbmitteln oder anderen Elementen versetzt oder dotiert sein. Als Farbmittel oder andere Elemente eignen sich beispielsweise organische oder anorganische Farbpigmente wie farbige Metalloxide, z.B. Magnetit, Chromoxid oder Farbpigmente wie z. B. Berliner Blau, Ultramarin, Bismutvanadat, Thenards Blau, oder aber organische Farbpigmente wie z.B. Indigo, Azopigmente, Phthalocyanine oder auch Karminrot oder Elemente wie z.B. Yttrium oder Antimon. Die äußere Schicht auf dem Substrat ist in einer bevorzugten Ausführungsform ein hochbrechendes Metalloxid. Diese äußere Schicht kann zusätzlich auf den oben genannten Schichtpaketen oder bei hochbrechenden Trägern Teil eines Schichtpaketes sein und z.B. aus TiO₂, Titansuboxiden, Fe₂O₃, SnO₂, ZnO, ZrO₂, Ce₂O₃, CoO, Co₃O₄, V₂O₅ Cr₂O₃ und/oder Mischungen davon, wie zum Beispiel Ilmenit oder Pseudobrookit, bestehen. TiO₂ ist besonders bevorzugt.
Die genannten beschichteten Substrate können eine oder mehrere, winkelabhängige Interferenzfarben zeigen. Sie können aber auch lediglich eine Absorptionsfarbe des Substrates bewirken bzw. eine Absorptionsfarbe zusätzlich zu einer oder mehreren, winkelabhängige Interferenzfarben.

Die Dicke der Metalloxid-, Metalloxidhydrat-, Metallsuboxid-, Metall-, Metallfluorid-, Metallnitrid-, Metalloxynitridschichten oder einer Mischung daraus beträgt üblicherweise 3 bis 500 nm und im Falle der Metalloxid-, Metalloxidhydrat-, Metallsuboxid-, Metallfluorid-, Metallnitrid-, Metalloxynitridschichten oder einer Mischung daraus vorzugsweise 5 bis 600 nm. Die Dicke der Metallschichten beträgt vorzugsweise 4 bis 100 nm.

Im Falle von Glasplättchen als plättchenförmigen Substraten sind diese ganz besonders bevorzugt mit einer SiO₂-Schicht beschichtet. Durch die SiO₂-Belegung wird die Glasoberfläche vor chemischer Veränderung wie Quellung, Auslaugen von Glasbestandteilen oder Auflösung in den aggressiven sauren Belegungslösungen geschützt. Während des Glühprozesses bei der Herstellung dieser Substrate kommt es im Fall der Glasplättchen an der Grenzfläche zwischen Glaskörper und aufgefälltem SiO₂ zu einem innigen Verbund der chemisch verwandten Materialien. Aufgrund der hohen Erweichungstemperatur gibt die aufgefällte SiO₂-Hülle den Substraten auch beim Glühen oberhalb von 700°C die erforderliche mechanische Stabilität. Auch die Haftung der auf die SiO₂-Schichten folgenden Beschichtung(en) ist sehr gut. Die Dicke der SiO₂-Schicht auf den Glasplättchen kann in Abhängigkeit vom gewünschten Effekt in weiten Bereichen variiert werden. Die Schicht weist Dicken von 5 - 350 nm, vorzugsweise von 5-150 nm, auf. Für die Steuerung von Glanz und Farbstärke sind Schichtdicken von 30- 100 nm bevorzugt. Die SiO₂-Schich kann auch mit Rußpartikeln, anorganischen Farbpigmenten, und/oder Metallpartikeln dotiert sein, sofern diese Dotierung an Luft oder unter Inertgas bei Temperaturen > 700°C stabil ist. Der Anteil an Dotiermittel in der SiO₂-Matrix beträgt dann 1 - 50 Gew.%, vorzugsweise 2-30 Gew.%, insbesondere 5 - 20 Gew.%. Auf die SiO₂-Schicht ist in einer besonders bevorzugten Ausführungsform eine Schicht eines hochbrechenden Metalloxids aufgebracht, insbesondere eine TiO₂-Schicht

Im Falle von sphärischen Substraten auf Basis von SiO₂ (z.B. Monosphere® oder Ronaspher®) weisen diese vorzugsweise eine Beschichtung mit Metalloxiden, insbesondere mit TiO₂ auf (z.B. Ronasphere® LDP).

Die erfindungsgemäßen Partikel weisen neben dem oben genannten plättchenförmigen, sphärischen oder nadelförmigen, beschichteten oder unbeschichteten Substrat bzw. Substrat-Gemisch eine Anion-bindende Schicht auf, insbesondere sind die Substrate mit der Anion-bindenden Schicht beschichtet. Vorzugsweise umfasst die Anion-bindende Schicht ein schichtförmiges Doppelhydroxid. Derartige Schichten werden auch als "layered double hydroxide"-Schichten (LDH-Schichten) bezeichnet.

Vorzugsweise ist das schichtförmige Doppelhydroxid (LDH) ein Doppelhydroxid der allgemeinen Formel

m²⁺₁₋ₓM³⁺ₓ(OH)₂(Zn⁻)_{x/n} · mH₂O

mit 0.2 < x < 0.33, wobei
M³⁺ ausgewählt ist aus Al³⁺, Cr³⁺, Fe³⁺, Ga³⁺, In³⁺, Y³⁺, La³⁺ und/oder Ce³⁺ und
M²⁺ ausgewählt ist aus Ba²⁺, Ca²⁺, Cu²⁺, Mg²⁺, Sr²⁺ und/oder Zn²⁺,
Zⁿ⁻ ein Gegenion der Metall-Salze und/oder ein Anion- oder ein Anionengemisch der Anion-bildenden organischen funktionellen Wirkstoffe bedeutet, dabei steht n für die Ladungszahl des Anions.
m ist ein stöchiometrischer Faktor und gibt den Gehalt an Kristallwasser im LDH an. Im Rahmen der vorliegenden Erfindung kann m beispielsweise 1-12 betragen, darüber hinaus aber auch andere Werte, die ganzzahlig oder nicht ganzzahlig sein können.

Beispiele der genannten Doppelhydroxide sind:
Mg_{0.67}Al_{0.33}(OH)₂(Zⁿ⁻)_{0.33/n} · mH₂O und Mg_{0.67}Fe_{0.33}(OH)₂(Zⁿ⁻)_{0.33/n} · mH₂O, Zn₀.₆₇Al_{0.33}(OH)₂(Zⁿ⁻)_{0.33/n} · mH₂O und Zn_{0.67}Fe_{0.33}(OH)₂(Zⁿ⁻)_{0.33/n} · mH₂O, Ca_{0.67}Al_{0.33}(OH)₂(Zⁿ⁻)_{0.33/n} · mH₂O und Ca_{0.67}Fe_{0.33}(OH)₂(Zⁿ⁻)_{0.33/n} · mH₂O. Insbesondere haben sich Ca_{0.67}Al_{0.33}(OH)₂(Zⁿ⁻)_{0.33/n} · mH₂O, Mg_{0.67}Al_{0.33}(OH)₂(Zⁿ⁻)_{0.33/n} mH₂O und Zn_{0.67}Al_{0.33}(OH)₂(Zⁿ⁻)_{0.33/n} · mH₂O als besonders vorteilhaft erwiesen.

Vorzugsweise ist M³⁺ = Al³⁺ oder Fe³⁺ und M²⁺ = Mg²⁺, Ca²⁺ oder Zn²⁺. Ganz besonders bevorzugt ist M³⁺ = Al³⁺. Die Schichtdicke der Anion-bindenden Schicht beträgt 0,5 - 500 nm, insbesondere 1 - 300 nm.

Die genannte Anion-bindende Schicht enthält einen oder mehrere Anion-bildende organische Wirkstoffe. Bei den organischen Wirkstoffen handelt es sich insbesondere um anionische oder Anion-bildende Arzneiwirkstoffe, Nahrungsergänzungsmittel, diagnostische Stoffe und Verbindungen und/oder kosmetische Wirkstoffe, das heißt es können auch Gemische der genannten Wirkstoffe vorliegen. Grundsätzlich gibt es bezüglich der funktionellen Wirkung der eingelagerten Wirkstoffe keine Begrenzung, solange sie nur anionisch sind, bzw. sich in Anionen überführen lassen. Insofern ist es möglich, organische Substanzen bzw. Substanzgemische auf dem Substrat zu fixieren, welche im Sinne einer pharmakologischen, und/oder einer hautschützenden, und/oder einer hautpflegenden, und/oder einer hautregenerierenden, und/oder einer antimikrobiellen, und/oder einer antifungiziden und/oder einer kosmetischen Wirksamkeit als Wirkstoffe fungieren. In einer bevorzugten Anwendung liegen diese Wirkstoffe mindestens zweifach anionisch geladen vor bzw. sind in mindestens zweifach anionisch geladene Moleküle überführbar. Solche Moleküle beinhalten mindestens zwei anionische oder in eine Anion überführbare funktionelle Gruppen. Solche können z.B. ausgewählt sein aus der Gruppe der Sulfonsäure (Sulfonat) und/oder Carbonsäure (Carboxylat) und/oder Schwefelsäuremonoester (Sulfat) und/oder Phenol (Phenolat) und/oder Phosphorsäuremono-, diester (Mono-, diphosphat), und/oder Phosphonate und/oder Enol (Enolat), und/oder Anionen der Imidsäure und/oder Anionen von Dithiolsäuren. Somit enthält der organische Wirkstoff eine oder mehrere, insbesondere mindestens zwei anionbildende Phenolat-, Enolat-, Carboxylat-, Sulfat-, Sulfonat-, Sulfinat-, Dithiolat-, Phosphat- und/oder Phosphit-Struktureinheiten. Im Falle dieser Wirkstoffe wird so ein eventuell unerwünschter Hautkontakt/ Hautpenetration (z.B. bei UV-Filtern) weitestgehend vermieden. Dieser Effekt der Immobilisierung des Wirkstoffes, Konservierungsstoffes oder Farbstoffes, insbesondere bei der Verwendung von mindestens zweifach ionisierbaren Stoffen, verstärkt im Falle der Stabilisierungsfunktion bei pH > 6 die Anionfixierung und drängt über eine so erhöhte Produktsicherheit unerwünschte Penetrationen zurück. Es kann so also bei pH > 6 eine nahezu vollständige Immobilisierung erreicht werden. Es kann aber auch, bei pH < 6, eine gezielte Freisetzung der Wirkstoffe eingestellt werden, die zu einer besseren Dosierung der Wirkstoffe führt.

Geeignete Anion-bildende Arzneiwirkstoffe sind beispielsweise:
1) Sedative Arzneistoffe: z.B. jene der Barbitursäurederivate, wie z.B. Natrium-Amobarbital (CAS: 64-43-7)
2) entzündungshemmende Anti-Rheumatika: z.B. jene der Essigsäurederivate, wie z.B. Indomethacin (CAS: 53-86-1), Acemetacin (CAS: 53164-05-9), Tolmetin (CAS: 26171-23-3), Diclofenac (CAS: 15307-86-5), Lonozolac (CAS: 53808-88-1) und/oder jene der Propionsäurederivate, wie z.B. Ibuprofen (CAS: 15687-27-1), Fenoprofen (CAS: 31879-05-7), Natrium-Naproxen (CAS: 26159-34-2), Ketoprofen (CAS: 22071-15-4)
3) anti-arteriosklerose B-Vitamine: z.B. Carnitin (CAS: 461-06-3), Aluminium-Nicotinsäure (CAS: 1976-28-9), Biotin (CAS: 58-85-5), Calcium-Pantothenat (CAS: 137-08-6), Aminobenzoesäure (CAS:150-13-0)
4) anti-arrythmische, anti-convulsante Nichtsteroide wie z.B. Voltaren/Diclofenac (CAS: 15307-86-5)
5) anti-arrythmische, beta-sympatholytische Substanzen der Klassse I, II, II und IV wie z.B. Bometolol (CAS: 65008-93-7) und BW-A-575-C (CAS: 103221-88-1)
6) β-Lactam-Antibiotika: z.B. jene der Penam-, Crabapenem-, Oxapenam-, Cephem-, Oxacephem- und monozyklischen β-Lactame, wie z.B. Carbenicillin (CAS: 4697-36-3), Amoxillin (CAS: 26787-78-0), Cefoxitin (CAS: 35607-66-0) und Ampicillin (CAS: 69-53-4)
7) Antibiotika aus der Familie der Penicilline, Aminopenicilline, Acylaminopenicilline, Carboxypenicilline oder Cephalosporine wie z.B. Tazobactam (CAS: 89786-04-9), Cloxacillin-Sulfon (CAS:76788-83-5), Sulbactam (CAS: 68373-14-8)
8) Antibiotika aus der Familie der Tetracycline wie z.B. Glycinmethyltetracyclin (CAS: 751-98-4), Lymecyclin (CAS: 992-21-2), Calcium-Chlortetracyclin (CAS: 57122-99-3), Apicyclin (CAS: 15599-51-6)
9) Anti-Coagulantien und Clotting-Faktor-Synthese-Inhibitoren aus der Familie der Coumarol-Derivate, wie z.B. Warfarin (CAS: 5543-58-8), Acenocoumarol (CAS: 152-72-7), 6-Hydroxywarfarin (CAS: 17834-02-5), 3-Hydroxy-Warfarin (CAS: 30992-81-5), Coumachlor (CAS: 81-82-3), 4-Hydroxy-Warfarin (CAS: 63740-78-3)
10) entzündungshemmende Prostaglandin-Antagonisten der Diphenylamin-Derivate, wie z.B. Clofenaminsäure (CAS: 4295-55-0), Aluminum-lufenamat (CAS: 16449-54-0), Natrium-Lobenzarit (CAS: 64808-48-6), Flutiazin (CAS: 7220-56-6), Araprofen (CAS: 15250-13-2)
11) Anti-Septika und Anti-Diabetika aus der Familie der Phenyl-Sulfonamide, wie z.B. Aristoplomb (CAS: 60662-80-8), Acrotiazol (CAS: 60595-59-7), BA-32641 (CAS: 92569-06-7), Alfasol (CAS: 38114-83-9), Carboxytolbutamid (CAS: 2224-10-4)
12) Entzündungshemmer, Analgetika, Collagenase-Hemmer und Keratolytische-Substanzen wie z.B. Salicylsäure (CAS: 69-72-7)
13) Antikonvulsanten z.B. jene der Valproat-Salze, wie z.B. Calcium-Valproat (CAS: 33433-82-8), Natrium-Valproat (CAS: 1069-66-5), Semi-Natrium-Valproat (CAS: 76584-70-8)
14) Anti-Asthmatika und Anti-Anaphylactika z.B. jene der Xanthin-Derivate, wie z.B. Acefyllin (CAS: 652-37-9), Natrium-Ablukast (CAS: 96565-55-8), Amlexanox (CAS: 68302-57-8), AH-7725 (CAS: 68302-57-8), Calcium-Nedocromil (CAS: 101626-68-0)
15) Cytostatika und Immunosuppressiva z.B. jene der Azathioprin-Derivate, wie z.B. Natrium-Azathioprin (CAS: 55774-33-9), Tiamiprin (CAS: 5581-52-2), Metazathioprin (CAS: 97746-12-8)
16) Corticosteroide und Progestogene z.B. jene der Prostaglandin-Derivate, wie z.B. Natrium-Methylprednisolon-Succinat (CAS: 2375-03-3), Natrium-Betamethasonphosphat (CAS: 151-73-5)
17) Viruzide z.B. jene der Guanosin-Derivate, z.B. 2'-Guanosin-mono-Phosphat (CAS: 130-50-7), 3'-Guanosin-mono-Phosphat (CAS: 82570-66-9), Aciclovir-phosphat (CAS: 66341-16-0), 2'-Cyclo-Guanosin-mono-Phosphat-NOR (CAS: 91516-85-7)
18) Thyroid-Hormone und Anti-Arteriosklerotika z.B. jene der Diphenylether-Derivate, wie z.B. Detrothyronin (CAS: 5714-08-9), Natrium-Levothyroxin (CAS: 55-03-8), Natrium-Dextrothyroxin (CAS: 137-53-1)
19) Analgetika, Entzündungshemmer, Anti-Pyretiks z.B. jene der Oxicam-Derivate, wie z.B. Meloxicam (CAS: 71125-38-7), Isoxicam (CAS: 34552-84-6), Piroxicam Olamin (CAS: 85056-47-9), Piroxicam-Phosphat (CAS: 82801-42-1), und/oder jene der Pyrazol-Derivate, wie z.B. Magnesium Metamizol (CAS: 6150-97-6) und Dibupyron (CAS: 1046-17-9) und/oder jene der Pyrazolidin-Derivate, wie z.B. Anthradion (CAS: 19854-90-1) und Sulfodethamedion (CAS: 53039-87-5)
20) Chelatoren, wie z.B. HBED (CAS: 35998-29-9), Calteridol (CAS: 132722-73-7), Cadystin-A (CAS: 86220-45-3), Natrium-Ditiocarb (CAS: 148-18-5), Muginein-Säure (CAS: 69199-37-7), CDTA (CAS: 13291-61-7), Calcium-Pentetat (CAS: 2531-75-1)
21) Protozoozide, z.B. jene der Artelinat-Derivate, wie z.B. Artesunat (CAS: 88495-63-0)
22) Prostaglandine für die Ulcus Therapie, wie z.B. Carbacyclin (CAS: 69552-46-1), Ataprost (CAS: 83997-19-7), Alfaprostol (CAS: 74176-31-1), Natrium-Beraprost (CAS: 88475-69-8), AY-16809 (CAS: 21269-28-3), alpha-PGF1 (CAS: 745-62-0).
Diese Beispiele sollen die Möglichkeiten exemplarisch erläutern, ohne die Auswahl einzuschränken.

Geeignete Anion-bildende Nahrungsergänzungsmittel, oder kosmetische Wirkstoffe, oder diagnostische Wirkstoffe sind beispielsweise die im weiteren Verlauf aufgeführten Bioflavonoide. Zudem eignen sich hierzu auch sämtliche Aminosäuren, wie z.B. Glutaminsäure und Asparaginsäure, ferner Aminosäuren mit Betainstruktur wie beispielsweise Ectoin, Hydroxyectoin oder Trimethylglycin. Ebenso sind anionbildende Vitamine und Vitaminderivate, insbesondere Phosphate, gemäß der vorliegenden Erfindung geeignet. Exemplarisch seien hier Vitamin C bzw. Vitamin-C-phosphat aufgeführt. Ferner können verwendet werden: Liponsäuren, Anionen von gesättigten- oder ungesättigten Fettsäuren, Retinsäure, Tocopherole (α, β, γ, δ), Vitamin B1 (z.B. Thiaminpyrophosphat), Vitamin B2, Vitamin B6 (Pyridoxin, Pyridoxal), Nicotinsäure, Pantothensäure, Biotin, Folsäure, Vitamin B12 (Cyanocobalamin), Nucleotide und Nucleoside bzw. deren Derivate wie z.B. Nicotinamid-adenin-dinucleotid (NAD bzw. NADH₂), Adenosintriphosphat (ATP), Adenosinmonophosphat (AMP), Adenosindiphosphat (ADP), 5'-Inosinmonophosphat (IMP), 5'-Guanosinmonophosphat (GMP), sowie Kreatin, Kreatinphosphat, Kreatinin. Ferner sind DNA- und/oder RNA-Bausteine bzw. DNA- und/oder RNA-Bruch- oder Teilstücke geeignet.

Als organische Wirkstoffe mit insbesondere antioxidativer Eigenschaft finden insbesondere Dicarbonsäuren (Oxalsäure, Bersteinsäure) und Hydroxycarbonsäuren (Milchsäure, Äpfelsäure, Weinsäure, Citronensäure) Verwendung.

Geeignete Anion-bildende diagnostische Stoffe und Verbindungen sind beispielsweise:
Röntgenkontrastmittel, wie z.B. locetaminsäure (CAS: 16034-77-8), lodocetylsäure (CAS: 54510-20-2), Meglumin-acetrizoat (CAS: 22154-43-4), lodohippurinsäure (CAS: 147-58-0), Natrium-Bunamiodat (CAS: 1923-76-8), Acetrizoinsäure (CAS: 85-36-9), aber auch Diagnostika für andere Anwendungen wie z.B. Dinitrochlorbenzol (CAS: 97-00-7), Gluceptin-Säure (CAS: 87-74-1), Natrium-Indigotindisulfonat (CAS: 860-22-0), locanlidin-Säure (CAS: 74855-17-7), Butedronin-Säure (CAS: 51395-42-7), Wofazurin (CAS: 7488-76-8), Upenazim (CAS: 95268-62-5), Vert-Sulfo-J (CAS: 519-76-6), Silber-Fluoreszin (CAS: 25931-86-6), Pankensan (CAS: 38219-60-2), Succimer (CAS: 304-55-2).
Diese Beispiele sollen die Möglichkeiten für gebundene anionische Nahrungsergänzungsmittel oder kosmetische Wirkstoffe oder diagnostische Substanzen lediglich exemplarisch erläutern, ohne aber die Auswahl einzuschränken.

Bei den kosmetischen Wirkstoffen handelt es sich insbesondere um pflegende und schützende Wirkstoffe. Beispiele für schützende Wirkstoffe sind UV-Filter, Stoffe mit antimikrobieller oder antifungizider Wirkung oder auch Konservierungsmittel.

Bevorzugt ist der funktionelle Wirkstoff ausgewählt aus der Klasse der UV-Filter. Bei UV-Filtern als funktionelle Wirkstoffe ist eine vollständige Immobilisierung der UV-Filter bevorzugt. Auf diese Weise wird ein möglicher Hautkontakt des UV-Filters vermieden, der vielfach nicht gewünscht ist. Weiterhin kann durch Auswahl entsprechender UV-Filter als Wirkstoffe mit entsprechend ausgewählten Substraten (z.B. ZnO) ein Breitband-UV-Filter bereit gestellt werden, der wegen der unterschiedlichen Absorptionseigenschaften von Wirkstoff und Substrat ein breites Absorptionsspektrum und damit einen verbesserten Schutz bietet.

Geeignete Anion-bildende oder anionische UV-Filter, die Ultraviolettlichtstrahlung absorbieren, können ausgewählt sein aus p-Aminobenz-imidazol-5-sulfonaten, 3-Imidazol-4-ylacrylaten, Salicylaten, p-Methoxy-cinnamaten, 2-Ethylhexyl-2-cyano-3,3-diphenylacrylaten, 3,3,5-Trimethyl-cyclohexyl-2-acetamidobenzoaten, p-Aminobenzoaten, Cinnamaten, 3,4-Dimethoxyphenylglyoxylaten, (2-Oxoborn-3-yliden)-p-xylol-2-sulfonaten, (2-Oxoborn-3-yliden)toluol-4-sulfonaten, Cyano-4-methoxycinnamaten oder 2-Phenylbenzimidazol-5-sulfonaten.

Diese Anionen sind, sofern sie im freien Zustand vorliegen, dafür bekannt, dass sie im Wellenlängenbereich von 290 bis 400 nm absorbieren. Alle Vertreter werden als verträgliche Materialien für Sonnenschutzstoffe angesehen.
Durch die Fixierung in der LDH-Schicht lässt sich das Absorptionsprofil genannter UV-Filter beibehalten, oder auch gezielt verschieben. Dadurch ist es letztlich auch möglich, die UV-Filter-Eigenschaften des Anions gezielt auf jene des Substrates abzustimmen. Besonders interessant und somit eine erfindungsgemäß bevorzugte Ausführungsform der vorliegenden Erfindung ist in diesem Zusammenhang die Möglichkeit UV-A- und UV-B-Filter-Eigenschaften in einem Produkt stabil miteinander zu verbinden.

Eine weitere Gruppe organischer Materialien, die für die erfindungsgemäßen Zwecke geeignet ist, sind jene, die eine schwach saure Funktionalität durch Einschluss eines phenolischen Protons oder eines anderen schwach sauren Protons in dem Molekül aufweisen. Es wurde als Teil der vorliegenden Erfindung gefunden, dass dieses Proton unter Bildung eines Anions, das in ein schichtenförmiges doppeltes Hydroxid eingeführt werden kann, entfernt werden kann. Anionen, abgeleitet von derartigen Verbindungen, können Absorptionsspektren aufweisen, die sich von der Stammverbindung wesentlich unterscheiden, jedoch zeigen diese Anionen nach Einmischen in ein schichtenförmiges doppeltes Hydroxid charakteristische Lichtabsorption zwischen 290 and 400 nm (UV-A-Bereich). In einer bevorzugten erfindungsgemäßen Ausführungsform ergibt sich ein Vorteil aus der Tatsache, dass diese Anionen, wenn sie in schichtenförmige doppelte Hydroxide eingemischt werden, derartige Spektren zeigen, so dass erhöhter UV-A-Schutz gegenüber jenem, der bei der Verwendung der Stammverbindungen in Abwesenheit des schichtenförmigen doppelten Hydroxids erhalten wird, entsteht. Eine bedeutende Gruppe derartiger Phenolverbindungen sind hydroxylierte Benzophenonderivate. Geeignete Diketonverbindungen (z.B. Dibenzoylmethane, wie z.B. Eusolex^{®} 9020), die in ihrer sauren Enolform vorliegen, können ebenfalls eingeschlossen sein. Beispiele von Verbindungen, von denen Anionen, angewendet in der erfindungsgemäßen bevorzugten Ausführungsform, abgeleitet sind, schließen nachstehende Materialien ein, sind jedoch nicht darauf beschränkt:

| CTFA-Name | Chemischer Name |
|---|---|
| Benzophenon-1 | 2,4-Dihydroxybenzophenon |
| Benzophenon-2 | 2,2',4,4'-Tetrahydroxybenzophenon |
| Benzophenon-3 | 2-Hydroxy-4-methoxybenzophenon |
| Benzophenon-4 | 2-Hydroxy-4-methoxybenzophenon-5-sulfonsäure |
| Benzophenon-5 | 2-Hydroxy-4-methoxybenzophenon-5-sulfonsäuremononatriumsalz |
| Benzophenon-6 | 2,2'-Dihydroxy-4,4-'dimethoxybenzophenon |
| Benzophenon-7 | 5-Chlor-2- hydroxybenzophenon |
| Benzophenon-8 | 2,2'-Dihydroxy-4-methoxybenzophenon |
| Benzophenon-9 | 2,2'-Dihydroxy-4,4'-dimethoxybenzophenon-3, 3'-disulfonsäuredinatriumsalz |
| Benzophenon-10 | 2-Hydroxy-4-methoxy-4'-methylbenzophenon |
| Benzophenon-12 | 2-Hydroxy-4-octoxybenzophenon |
| Homosalat | Salicylsäurehomomenthylester |
| Octylsalicylat | Salicylsäure-2-ethylhexylester |

Eine weitere Verbindung, die Zwischenschichtanionen bereitstellen kann, ist Butylmethoxydibenzoylmethan, erhältlich als PARSOL 1789 von Givaudan Corp.
Ebenfalls in dieser erfindungsgemäßen Form eingeschlossen sind anionische Spezies, abgeleitet von Pongomol, ein substituiertes 1,3-Diketon, dessen systematischer Name 1-(4-Methoxy-5-benzofuranyl)-3-phenyl-1,3-propandion ist. Es weist eine ultraviolette Absorptionsbande innerhalb des Bereiches von 250 bis 500 nm und einen Extinktionskoeffizienten von 5000 bis 70 000 auf. Dieses Diketon ist genauer beschrieben in der US-Patentschrift 5,152,983 deren Offenbarung durch diesen Hinweis in die Beschreibung aufgenommen wird.
Unter diesen Stoffen haben sowohl Benzophenon-4 als auch Benzophenon-9 eine stark saure Funktionalität, verliehen durch eine Sulfonatgruppe, und eine schwach saure Funktionalität, verliehen durch das phenolische Proton. Für diese Materialien (und für Benzophenon-5, nämlich dem Mononatriumsalz von Benzophenon-4), können mehrfach anionische Formen des Stoffes hergestellt werden und in die Hydroxid-Fixierungsschicht eingeführt werden. Beispielsweise kann Benzophenon-4 sowohl als Mono- als auch als Dianion auf dem Substrat fixiert werden. Sowohl monoanionische als auch dianionische Formen dieses Materials und beliebige Kombinationen davon, die in die schichtenförmigen doppelten Hydroxide eingeführt werden, können für Sonnenschutzmittel geeignet sein und werden als innerhalb des Schutzbereichs der Erfindung liegend angesehen.

Insbesondere bevorzugt sind die UV-Filter ausgewählt aus:
Phenylbenzimidazoliumsulfonsäure (CAS 27503-81-7; Handelsname Eusolex 232) Benzophenon-4 (CAS 4065-45-6; Handelsname Uvinul-MS-40 (BASF)) Benzylidencamphersulfonsäure (CAS 56039-58-8; Handelsname Mexoryl SL (Chimex/Loreal)), Terephthaliden-di-campher-sulfonsäre (CAS 90457-82-2; Handelsname Mexoryl SX (Chimex, Loreal)), Dinatriumphenyl-dibenzimidazoliumtetrasulfonate (CAS 180898-37-7; Handelsname Neo Heliopan AP (Symrise)).

Geeignete Anion-bildende Konservierungsmittel sind Benzoesäure, Salicylsäure, p-Hydroxybenzoesäure, p-Hydroxybenzoesäureester (PHB-Ester), Sorbinsäure, Propionsäure, Essigsäure, Sulfite, Diethyldicarbonat, Dimethyldicarbonat, Nitrit, Nitrat, anionbildende Antibiotika, o-Phenylphenol.
Ebenfalls als Anion-bildende Wirkstoffe geeignet sind Bioflavonoide, die sich aufgrund ihrer Grundstruktur in die Gruppen der Chalkone, Aurone, Flavanone, Flavan-3-ole (Catechine), Flavone, Isoflavone, Flavan-3,4-diole (Leukoanthocyanidine), Flavonole (3-Hydroxy-flaven-4-on) oder Flavanonole zuordnen lassen.

Beispielhaft sollen folgende Flavonoide angeführt werden: 5-Hydroxy-7,4'-dimethoxyflavon-8-sulfat, 7,8-Dihydroxyflavon, Luteolin (Flavone); Catechin, Epicatechin, EpiGalloCatechinGallat (EGCG, TEAVIGO^{®} DSM) (Flavan-3-ole bzw. Flavan-3-ol-derivate); Kämpferol (Flavonol); Taxifolin (Flavanonol).

Bevorzugte Flavonoide leiten sich von den Gruppen der Flavonole, Flavonol-o-glycoside oder Flavonol-o-glycosid-enthaltenden Extrakte ab. Flavonoide kommen meist als lösliche Glykoside im Zellsaft der Pflanze vor. Die bevorzugten Flavonoide umfassen auch Aglyka (zuckerfreie Strukturen) und Aglyka-Konjugate. Mögliche Aglyka-Konjugate sind Hydroxyl-Derivate, wobei die Hydroxylgruppen ganz oder teilweise alkyliert, methyliert, glycyliert, sulfatiert oder verestert sind. Neben Hydroxylderivaten kommen auch C-Derivate als Aglyka-Konjugate in Frage.

Besonders bevorzugt ist für die Gruppe der Flavonole das Aglykon Quercetin. Bei der Gruppe der Flavonol-o-glycoside sind die Flavonol-3-glycoside wie Rutin, α-Glucosylrutin, Tilirosid, Isoquercetin, Rutinsulfat, Trishydroxyethylrutin (Troxerutin) sowie deren Sulfate und Phosphate besonders bevorzugt. Der Begriff "Rutinsulfat" umfasst Mono-, Di-, Tri-, Tetra- oder Polysulfate des Rutins bzw. Gemische dieser Rutinsulfate. Der Begriff "Troxerutin" umfasst Mono-, Di-, Tri- Tetra- oder Polyethoxylate des Rutins bzw. Gemische dieser Rutinethoxylate. Auch Flavonol-7- und -8-glycoside können verwendet werden.

Die beschriebenen Substanzklassen sind nur beispielhaft zu verstehen und sollen die vorliegende Erfindung lediglich erläutern, ohne sie zu begrenzen. Die verschiedenen Wirkstoffe können natürlich miteinander kombiniert werden, das heißt es können Mischungen mehrerer funktioneller Wirkstoffe auf dem Substrat fixiert werden.

Die dadurch fixierte Menge an Wirkstoff beträgt zwischen 0.001 und 50 Gew.-%, bezogen auf das Gesamtpigment. Bevorzugt werden zwischen 0.5 und 20.0 Gew.-%, insbesondere zwischen 1.0 und 10.0 Gew.-% fixiert.

In einer besonders bevorzugten Ausführungsform der vorliegenden Erfindung enthält die Anion-bindende Schicht neben dem Wirkstoff zusätzlich Anion-bildende organische, anorganische und/oder metallorganische Farbmittel. Damit werden multifunktionelle Partikel erhalten, die neben der funktionellen Wirkung des Wirkstoffes zusätzlich einen Farbeffekt zeigen. Auf diese Weise werden zwei voneinander unabhängige Eigenschaften miteinander kombiniert. Dies hat den Vorteil für den Anwender, dass funktionelle Wirkung und Farbeffekt nicht mehr über getrennt zuzugebende Komponenten in ein Anwendungssystem eingebracht werden müssen, sondern dies durch Zugabe nur einer Komponente erfolgen kann. Dies vereinfacht die Herstellung von Formulierungen und vergrößert die Variabilität für die Anwendungen. Durch die gleichzeitige Zugabe von gleich mehreren Funktionen in die Formulierung in einem Arbeitsschritt lassen sich über die kürzere Formulierungszeit beim Kunden zudem Einsparungen erzielen. Dies wird noch dadurch verstärkt, dass die organischen Wirkstoffe/Farbmittel bzw. Gemische daraus durch die Fixierung auf einem Substrat bereits in einer Art vordispergiertem Zustand vorliegen. Die nicht fixierten organischen Wirkstoff/Farbmittel sind nämlich bekanntermaßen oftmals schwer zu dispergieren. Durch die erfindungsgemäßen Partikel entfällt hingegen dieser Zeit- und Energie-aufwendige Dispergierschritt bzw. tritt nur in sehr stark verkürzter Form auf.

Besonders intensive Farbeindrücke erreicht man, wenn die Interferenzfarbe des eingesetzten Substrates mit der Absorptivfarbe des Farbmittels übereinstimmt. Unterscheidet sich die Interferenzfarbe des Substrates von der Absorptivfarbe des Farbmittels, so erzielt man attraktive Multicolor-Effekte, welche dann noch mit der Aktivität des funktionellen Wirkstoffs kombiniert sind.

Solche Farbmittel können sein: von organischen Farbstoffen oder Pigmenten bzw. von Farbstoff- oder Pigmentvorstufen abgeleitete Anionen oder anorganische bzw. organische Anionen oder Radikalanionen bzw. farbige Heteropolyanionen bzw. ein Gemisch aus den zuvor genannten Farbmitteln.

Für Nicht-Kosmetik-Anwendungen können als Farbmittel prinzipiell alle anionischen bzw. anionbildenden Farbstoffe eingesetzt werden. Geeignet sind dabei insbesondere C.I. Acid-Farbstoffe wie z.B.: C.l. Acid Yellow 13, C.l. Acid Yellow 17, C.I. Acid Yellow 23, C.I. Acid Yellow 25, C.I. Acid Yellow 36, C.l. Acid Yellow 38, C.I. Acid Yellow 42, C.l. Acid Yellow 44, C.I. Acid Yellow 56, C.I. Acid Yellow 65, C.I. Acid Yellow 76, C.I. Acid Yellow 127, C.l. Acid Orange 7, C.l. Acid Orange 10, C.I. Acid Orange 19, C.l. Acid Orange 65, C.I. Acid Orange 67, C.l. Acid Red 1, C.l. Acid Red 13, C.I. Acid Red 14, C.I. Acid Red 32, C.I. Acid Red 37, C.l. Acid Red 38, C.l. Acid Red 42, C.l. Acid Red 88, C.l. Acid Red 119, C.I. Acid Red 131, C.I. Acid Red 138, C.l. Acid Red 154, C.l. Acid Red 249, C.l. Acid Red 299, C.l. Acid Violet 14, C.l. Acid Violet 42, C.l. Acid Violet 43, C.I. Acid Blue 25, C.I. Acid Blue 40, C.l. Acid Blue 43, C.l. Acid Blue 62, C.l. Acid Blue 92, C.l. Acid Blue 113, C.l. Acid Blue 117, C.l. Acid Blue 129, C.l. Acid Green 1, C.l. Acid Green 25, C.l. Acid Green 41, C.l. Acid Black 1, C.l. Acid Black 24, C.I. Acid Black 26, C.I. Acid Black 48, C.I. Acid Black 210, C.I. Acid Black 234, C.I. Acid Brown 14, C.I. Acid Brown 20.
Darüber hinaus sind auch C.I. Reaktiv-Farbstoffe geeignet wie z.B.: C.I. Reactive Yellow 4, C.I. Reactive Yellow 17, C.I. Reactive Orange 1, C.I. Reactive Red 8, C.I. Reactive Red 12, C.I. Reactive Red 23, C.I. Reactive Blue 15, C.I. Reactive Blue 19, C.I. Reactive Blue 216, C.I. Reactive Black 5, C.I. Reactive Black 8, C.I. Reactive Black 31.
Ferner sind geeignet C.I. Direkt-Farbstoffe wie z.B.:
C.I. Direct Yellow 12, C.I. Direct Yellow 27, C.I. Direct Yellow 29, C.I. Direct Yellow 50, C.I. Direct Yellow 86, C.I. Direct Orange 26, C.I. Direct Red 23, C.l. Direct Red 75, C.l. Direct Red 76, C.l. Direct Red 79, C.l. Direct Red 80, C.l. Direct Red 81, C.l. Direct Red 250, C.l. Direct Blue 78, C.l. Direct Blue 86, C.l. Direct Blue 93, C.l. Direct Blue 106, C.l. Direct Green 26, C.l. Direct Black 19, C.l. Direct Black 22, C.l. Direct Black 51, C.l. Direct Black 150, C.l. Direct Black 151, C.l. Direct Black 166, C.l. Direct Black 168. Schließlich können auch C.l. Mordant-Farbstoffe, wie z.B.: C.l. Mordant Yellow 1, C.l. Mordant Yellow 5, C.l. Mordant Yellow 30, C.l. Mordant Red 7, C.l. Mordant Red 19, C.l. Mordant Red 30, C.l. Mordant Blue 7, C.l. Mordant Blue 13, C.l. Mordant Black 3, C.l. Mordant Black 9, C.l. Mordant Black 11, C.l. Mordant Brown 33, C.l. Mordant Brown 48, aber auch C.l. Solubilised Sulphur Red 11 und Fluoreszenz-Farbstoffe wie z.B.: C.l. Basic Yellow 40, C.l. Basic Red 12, C.l. Solvent Yellow 94 eingesetzt werden.

Bevorzugt eingesetzt werden die Anionen der organischen Farbstoffe und Pigmente bzw. deren Vorstufen und Gemische, welche in Kosmetik-Anwendungen zugelassen sind. Beispiele sind: FD&C Yellow 5 (Tartrazin), FD&C Yellow 6 (Sunset Yellow FCF), FD&C Yellow 10, FD&C Red 3 (Erythrosin), FD&C Red 6 (Litholrubin B), FD&C Red 7 (Litholrubin BN), FD&C Red 21, FD&C Red 27, FD&C Red 28 (Floxine B), FD&C Red 33, C.I. Natural Red 33, FD&C Red 36, FD&C Red 40, Carmine, FD&C Blue 1 (Brilliant Blue FCF), C.I. Natural Green 3 (E141), FD&C Blue, FD&C Black 1 (Brilliant Black).

Es besteht insbesondere auch die Möglichkeit Gemische von mindestens 2 Farbmittelanionen über die Anion-bindende Schicht auf dem Substrat zu fixieren. Hierbei ergeben sich unzählige Farbvarianten. Ebenfalls sind der prozentualen Zusammensetzung der Farbmittel-Gemische keinerlei Beschränkungen auferlegt.

Die beschriebenen Farbmittel sind nur beispielhaft zu verstehen und sollen die vorliegende Erfindung lediglich erläutern, ohne sie zu begrenzen. Die verschiedenen Substrate können natürlich auch mit anderen anionischen/anionbildenden organischen oder anorganischen Farbmitteln kombiniert werden.

Der Anteil der Anion-bildenden organischen, anorganischen und/oder metallorganischen Farbmittel bzw. Gemischen daraus beträgt 0.01 bis 30 Gew.-%, bezogen auf das Gesamtpigment, insbesondere 0.5 bis 10 Gew.-%.

In einer besonders bevorzugten und erläuternden Ausführungsform der vorliegenden Erfindung enthält die anionbindende Schicht bestehend aus Ca_{0.67}Fe_{0.33}(OH)₂(Zⁿ⁻)_{0.33/n} · mH₂O einen UV-Filter und/oder hautaktive Substanzen. Das Komposit ist aufgefällt auf ein plättchenförmiges Interferenzpigment. Damit werden multifunktionelle Partikel erhalten, welche besonders für den Einsatz als sogenannter skin-corrector in kosmetischen Anwendungen geeignet sind. Dabei bewirken die Interferenzfarbe des Substrates und die hautfarbene Absorptionsfarbe der anionbindenden Schicht eine unmittelbar wahrnehmbare, optische Korrektur von farblichen Haut-Inhomogenitäten und die im Komposit gebundenen Substanzen sorgen für einen vorteilhaften Zusatzeffekt der eben solche Hautverfärbungen nicht nur optisch, sondern aktiv unterbindet oder zumindest abschwächt.

In einer weiteren Ausführungsform der vorliegenden Erfindung ist auf den Partikeln zusätzlich eine stabilisierende anorganische und/oder organische Beschichtung aufgebracht. Diese Nachbeschichtung erhöht, in Abhängigkeit vom Einsatzgebiet, die Licht-, Wasser- und Wetterstabilität. Dabei wird auch die Ausblutbeständigkeit des Produktes weiter erhöht. Beispiele für derartige Beschichtungen finden sich z.B. in DE 22 15 191, DE 31 51 354, DE 33 34 598, EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 EP 0 090 259, EP 0 634 459, WO 99/57204, WO 96/32446, WO 99/57204, US 5,571,851, WO 01/92425. oder EP 0 465 805, deren Offenbarung hiermit unter Bezugnahme mit eingeschlossen ist. Als anorganische Materialien für die Nachbeschichtung eignen sich die Oxide und/oder Oxidhydrate von Al, Si, Zr, Ce, Zn, Fe und/oder Mischungen hieraus, vorzugsweise werden die Oxide und/oder Oxidhydrate von Al, Ce, Zn, Zr und/oder Si eingesetzt. Die genannten Schichten können als einzelne Schichten der jeweiligen Oxide und/oder Oxidhydrate aber auch als Mischschichten vorliegen. Darüber hinaus können neben den alleinig abgeschiedenen Oxiden auch Mischungen von Oxiden mit Sulfaten, Phosphaten und/oder Boraten eingesetzt werden. Beispiele für Sulfate sind ZnSO₄, CaSO₄, für Phosphate AlPO₄, CePO₄ und für Borate AlBO₄.

Schichten dieser Materialien zeichnen sich durch hohe Transparenz, fehlende oder geringe Eigenfarbe und hohen Glanz aus, so dass die koloristischen Eigenschaften der Partikel nicht verändert werden. Insgesamt sind die jeweiligen Anteile für die zusätzliche stabilisierende Beschichtung so auszuwählen, dass die optischen Eigenschaften der erfindungsgemäßen Partikel nicht wesentlich beeinflusst werden.

Die gegebenenfalls aufgebrachte organische Beschichtung agiert als Kupplungsreagenz und kann aus Organosilanen, -aluminaten, -titanaten und/oder zirkonaten der allgemeinen Formel

X₄₋ₙ₋ₘZ-Rₙ(-B-Y)ₘ

mit X = OH, Halogen, Alkoxy, Aryloxy
Z = Si, Al, Ti, Zr
R = Alkyl, Phenyl oder Wasserstoff
B = organische, zumindest bifunktionelle Gruppe (Alkylen,
Alkylenoxyalkylen)
Y = Amino-, substituierte Amino-, Hydroxy-, Hydroxyalkyl-, Siloxan-, Acetoxy, Isocyanat-, Vinyl-, Acryloyl-, Epoxy-, Epoxypropyloxy-, Imidazol- oder Ureidogruppe
n, m = 0,1,2,3 mit n+m ≤ 3 bestehen.

Die Kupplungsreagenzien bestehen aus einer Ankergruppe (X₄₋ₙ₋ₘZ), die an die Oberfläche bindet, wenigstens einer hydrophoben Gruppe (R,B) sowie einer oder mehrerer funktioneller Gruppe (Y). Vorzugsweise handelt es sich bei den Kupplungsreagenzien um Verbindungen mit Z = Si. Bevorzugt besteht die Ankergruppe aus Alkoxysilanen, die durch hydrolytische Reaktionsbedingungen in entsprechende Hydroxygruppen überführt werden können. Letztere können an die Oberfläche der Partikel binden und die Verankerung über Sauerstoffbrücken bewirken. Darüber hinaus können auch Mischungen verschiedener Kupplungsreagenzien eingesetzt werden, die als Mischung oder einzeln aufgebracht werden können.

Durch die Wahl geeigneter funktioneller Gruppen kann die organische Beschichtung dem Einsatzmedium angepasst werden. Darüber hinaus können durch Reaktion der funktionellen Gruppen mit entsprechenden Funktionalitäten in den Applikationsmedien zusätzliche Bindungen zwischen Pigment und Medium über das Kupplungsreagenz erzeugt werden. In einer besonderen Ausführungsform wird die Oberfläche der erfindungsgemäßen Pigmente mit einer dem Einsatzmedium angepassten Kombination von organischen Funktionalitäten modifiziert. Hierzu eignet sich auch der Einsatz von Mischungen verschiedener Kupplungsreagenzien innerhalb der organischen Beschichtung. Die Hydrophobie der Pigmentoberfläche kann durch Integration von alkylhaltigen Kupplungsreagenzien wie z. B. Alkylsilanen angepasst werden. Neben den Organosilanen ist auch der Einsatz ihrer Hydrolysate sowie homogener und heterogener Oligomere und/oder Polymere bevorzugt, die ebenfalls alleinig oder in Kombination mit Silanen, Zirkonaten, Aluminaten, Zirkonaluminaten und/oder Carboxyzirkonaluminaten als organische Beschichtung eingesetzt werden können. Im Besonderen bevorzugt ist eine organische Beschichtung mit Mischungen verschiedener Kupplungsreagenzien, insbesondere mit voneinander unterschiedlichen funktionellen Gruppen Y, die eine besondere Anwendungsbreite gewährleistet.

Beispiele für Organosilane sind Propyltrimethoxysilan, Propyltriethoxysilan, Isobutyltrimethoxysilan, n-Octyltrimethoxysilan, i-Octyltrimethoxysilan, n-Octyltriethoxysilan, n-Decyltrimethoxysilan, Dodecyltrimethoxysilan, Hexadecyltrimethoxysilan, Vinyltrimethoxysilan, vorzugsweise n-Octyltrimethoxysilan und n-Octyltriethoxysilan. Als oligomere, alkoholfreie Organosilanhydrolysate eignen sich unter anderem die unter dem Handelsnamen "Dynasylan^{®}" von der Fa. Sivento vertriebenen Produkte, wie z. B. Dynasylan HS 2926, Dynasylan HS 2909, Dynasylan HS2907, Dynasylan HS 2781, Dynasylan HS 2776, Dynasylan HS 2627. Darüber hinaus eignet sich oligomeres Vinylsilan als auch Aminosilanhydrolysat als organische Beschichtung. Funktionalisierte Organosilane sind beispielsweise 3-Aminopropyltrimethoxysilan, 3-Methacryloxytrimethoxysilan, 3-Glycidyloxypropyltrimethoxysilan, beta-(3,4-Epoxycyclohexyl)-ethyltrimethoxysilan, gamma-Isocyanatopropyltrimethoxysilan, 1,3-bis(3-glycidoxypropyl)-1,1,3,3,-tetramethyldisiloxan, Ureidopropyltriethoxysilan, bevorzugt sind 3-Aminopropyltrimethoxysilan, 3-Methacryloxytrimethoxysilan, 3-Glycidyloxypropyltrimethoxysilan, beta-(3,4-Epoxycyclohexyl)-ethyltrimethoxysilan, gamma-Isocyanatopropyltrimethoxysilan. Beispiele für polymere Silansysteme sind in WO 98/13426 beschrieben und werden z. B. von der Fa. Sivento unter dem Warenzeichen Hydrosil^{®} vertrieben.

Die Menge der organischen Beschichtung beträgt zwischen 0.2 und 5 Gew.-%, bezogen auf das Pigment, vorzugsweise 0.5 bis 2 Gew.-%.

Ein weiterer Gegenstand der vorliegenden Erfindung sind Verfahren zur Herstellung der erfindungsgemäßen Partikel wobei eine Suspension von Substrat(en), Metallkation-Salzen, Wirkstoff- bzw. Farbmittelsalzen bzw. Gemischen dieser Salze (nachfolgend kurz als "Wirkstoff-Salz" benannt), sowie Laugen und/oder Harnstoff in einem Lösungsmittel oder Lösungsmittelgemisch bei einer Temperatur von 50-120°C gerührt wird, so dass sich auf dem Substrat die Anion-bindende Schicht bildet, wobei die Anion-bindende Schicht Anion-bildende organische Wirkstoffe enthält, anschließend das Produkt abgetrennt, gewaschen, getrocknet und gegebenenfalls gesiebt wird.

Im einfachsten Falle werden das Substrat, die Metallkation-Salze und Wirkstoff-Salz(e) zusammen mit Lauge und/oder Laugenvorstufen in einer Suspension vorgelegt und im Falle von schichtförmigen Doppelhydroxiden abhängig von der Art des LDH und des eingesetzten Wirkstoff-Salzes bei einer Temperatur von 10-120°C für 2 bis 48 Stunden gerührt. Nach Abkühlen der Reaktionssuspension wird das Produkt abgesaugt und mit einem geeigneten Lösungsmittel gewaschen, bis das Filtrat nahezu wirkstofffrei abläuft. Der Filterkuchen wird anschließend bei 50-180°C getrocknet und gegebenenfalls auf die gewünschte Feinheit gesiebt.

In einer weiteren Ausführung kann eine Lösung der Metallkation-Salze zu einer Suspension aus Substrat(en), Wirkstoff-Salzen sowie Laugen und/oder Laugenvorstufen zugegeben werden. Dies bedeutet im einfachsten Falle, dass das Substrat, Lauge und/oder Laugenvorstufen und Wirkstoff-Salz(e) in einer Suspension vorgelegt werden. Bei Raumtemperatur wird anschließend eine Lösung der Metallkation-Salze zugetropft. Nach Beendigung der Zugabe wird das Reaktionsgemisch auf 50-100°C geheizt und abhängig von der Art des LDH und des Wirkstoffs bei dieser Temperatur für 2 bis 48 Stunden gerührt. Nach Abkühlen der Reaktionssuspension wird das Produkt abgesaugt und mit einem geeigneten Lösungsmittel gewaschen, bis das Filtrat nahezu wirkstofffrei abläuft. Der Filterkuchen wird anschließend bei 50-180°C getrocknet und gegebenenfalls auf die gewünschte Feinheit gesiebt.

Als Laugen eignen sich wässrige Lösungen von NaOH, KOH oder NH₃, aber auch Laugenvorstufen, wie z.B. Harnstoff, welche im ReaktionsMedium, z.B. durch Hydrolyse, erst die eigentliche Lauge freisetzen. Der pH-Wert bei der Umsetzung (gemeint ist hier der pH-Wert-Verlauf während der gesamten Reaktion) liegt üblicherweise im Bereich von 2 bis 13, insbesondere von 3 bis 11.

Die für die Bildung der LDH-Schicht geeigneten Metallkationen sind aus der Fachliteratur bekannt. Als Metallkation-Salze eignen sich grundsätzlich alle löslichen Salze mit Al³⁺, Cr³⁺, Fe³⁺, Ga³⁺, ln³⁺, Y³⁺, La³⁺, Ce³⁺, Ba²⁺, Ca²⁺, Cu²⁺, Mg²⁺, Sr²⁺ und/oder Zn²⁺ Kationen, aus denen die Anion-bindende Schicht bestehend aus einem schichtförmigem Doppelhydroxid hergestellt werden kann. Insbesondere handelt es sich bei den genannten Metallkation-Salzen um die entsprechenden Halogenide, insbesondere Chloride, Bromide, Iodide. Es sind aber auch die Sulfate und Nitrate der Metallkationen geeignet. Ganz besonders bevorzugt werden die entsprechenden Chloride und Nitrate eingesetzt.

In einer alternativen, 2-stufigen Ausführungsform zur Herstellung der erfindungsgemäßen Multifunktionspartikel wird eine Suspension des Substrates/Substrat-Gemisches, Metallkation-Salze sowie Laugen und/oder Harnstoff in einem Lösungsmittel oder Lösungsmittelgemisch bei einer Temperatur von 10-120°C gerührt, so dass sich auf dem Substrat die Anion-bindende Schicht bestehend aus einem schichtförmigem Doppelhydroxid bildet, danach wird das Zwischenprodukt abgetrennt, gewaschen, getrocknet bei 50-300°C und gegebenenfalls gesiebt. Dieses mit LDH belegte Substrat wird anschließend zu einer Lösung des Anion-bildenden organischen funktionellen Wirkstoffs unter Rühren hinzu gegeben. Bei dieser Verfahrensvariante kommt es zu einem Ionenaustausch, wobei das Anion Zⁿ⁻ des Anion-bindenden Doppelhydroxids gegen den Anion-bildende Wirkstoff ausgetauscht wird. Nach Abkühlen der Reaktionssuspension wird das Produkt abgesaugt und gewaschen. Der Filterkuchen wird bei 40-70°C getrocknet. Das trockene Hybridpigment kann anschließend eventuell vermahlen und/oder gesiebt werden.

Bei der letztgenannten Methode sollten als Metallsalze vorzugsweise entsprechende Chloride oder Nitrate eingesetzt werden, da diese beiden Anionen im anschließenden Schritt am Schnellsten ausgetauscht werden. Diese 2-stufige Methode eignet sich insbesondere auch gut für temperaturempfindliche Wirkstoffe, weil im Vergleich zu den anderen Verfahren der Wirkstoff während der Synthese nicht längerer Zeit hohen Temperaturen ausgesetzt wird und das wirkstoffhaltige Endprodukt zudem bei deutlich niedrigerer Temperatur getrocknet werden kann.

Bei dieser 2-stufigen Synthese-Variante hat es sich darüber hinaus auch als vorteilhaft erwiesen, das Zwischenprodukt mit der auf dem Substrat gebildeten Anion-bindenden Schicht bestehend aus einem schichtförmigem Doppelhydroxid vor Zugabe zu der Lösung der Anion-bildenden organischen Wirkstoffe bei Temperaturen von 300-600°C zu kalzinieren. Dabei wird das mit LDH belegte Substrat durch Kalzinieren bei 300-600°C zunächst in ein LDO-belegtes Substrat umgewandelt. Das LDO ("layereddouble-oxide") bildet anschließend im wässrigen bzw. wasserhaltigen Medium wieder die LDH-Struktur zurück und ermöglicht dabei eine leichtere und vollständigere Interkalation.

In einer alternativen Ausführungsform des zuvor beschriebenen 2-stufigen Verfahrens kann auch zunächst eine Suspension des mit LDH bzw. LDO belegten Substrates hergestellt werden, zu der dann bei 20-70°C unter Rühren eine Lösung des Wirkstoff-Anions gegeben wird. Das Gemisch lässt man üblicherweise 2 bis 48 h bei dieser Temperatur rühren. Nach beendeter Fixierung des Wirkstoff-Anions wird das Produkt abgesaugt und mit einem geeigneten Lösungsmittel gewaschen, bis das Filtrat nahezu wirkstofffrei abläuft. Der Filterkuchen wird anschließend bei 40-70°C getrocknet und gegebenenfalls auf die gewünschte Feinheit gesiebt.

Die oben genannten Verfahren eignen sich sowohl für die Herstellung von Partikeln mit Wirkstoffen als auch für die bevorzugte Herstellung von Partikeln mit einer Kombination von Wirkstoffen und Farbmitteln. Die Farbmittel können dabei von Beginn an in einer Mischung mit den Wirkstoffen vorliegen oder nachträglich hinzugefügt werden. Insbesondere ist es auch möglich zunächst ein Farbmittel- oder Wirkstoffenthaltendes Partikel herzustellen und anschließend in einem weiteren Schritt die jeweils andere Komponente mittels Ionenaustausch, wie oben beschrieben, in die Anion-bindende Schicht einzulagern.

Darüber hinaus kann in einem ebenfalls erfindungsgemäßen Verfahren zusätzlich als äußere Schicht eine anorganische und/oder organische Beschichtung aufgebracht werden. Beispiele für derartige Beschichtungsverfahren finden sich unter anderem in EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465 805. Beispiele für anorganische und/oder organische Beschichtungen sowie die damit verbundenen Vorteile sind bereits vorab beim Aufbau der erfindungsgemäßen Pigmente beschrieben worden. Der Verfahrensschritt der Aufbringung der organischen Beschichtung kann direkt an die anderen Schritte des erfindungsgemäßen Verfahrens angeschlossen werden. Die Aufbringung der Kupplungsreagenzien erfolgt in Lösung bei Temperaturen oberhalb von 60°C, vorzugsweise oberhalb von 70°C. Als Lösungsmittel eignen sich organische Lösungsmittel, Wasser oder Mischungen hieraus, bevorzugt wird Wasser verwendet. Die für die Aufbringung der organischen Beschichtung nötige Reaktionszeit liegt bei mindestens 5 Minuten, vorzugsweise erfolgt sie über einen Zeitraum von 10 bis 90 Minuten, kann aber auch beliebig verlängert werden. Das erhaltene Pigment wird nach für den Fachmann gebräuchlichen Methoden aufgearbeitet und isoliert, z. B. durch Filtration, Trocknung und Siebung.

Die erfindungsgemäßen Partikel sind vielfältig einsetzbar. Demgemäß ist die Verwendung der erfindungsgemäßen Partikel in Kosmetika, Pharmazeutika, Formulierungen, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten ebenfalls Gegenstand der vorliegenden Erfindung.

Im Falle von Kosmetika eignen sich die erfindungsgemäßen Hybridpigmente besonders für Produkte und Formulierungen der dekorativen und pflegenden Kosmetik, wie z.B. Salben, Cremes, Pasten, Nagellacke, farbgebende Puder, Lippenstifte oder Lidschatten, Seifen, Zahnpasten, Selbstbräunungsformulierungen etc. Selbstverständlich können die erfindungsgemäßen Hybridpigmente in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z.B. Verdicker und rheologische Zusatzstoffe wie etwa Bentonite, Hektorite, Siliziumdioxid, Ca-Silikate, Gelatine, hochmolekulare Kohlenhydrate und/oder oberflächenaktive Hilfsmittel, etc. Die erfindungsgemäße Hybridpigmente enthaltenden Formulierungen können dem lipophilen, hydrophilen oder hydrophoben Typ angehören. Bei heterogenen Formulierungen mit diskreten wässrigen und nicht-wässrigen Phasen können die erfindungsgemäßen Partikel in jeweils nur einer der beiden Phasen enthalten oder auch über beide Phasen verteilt sein.

Die pH-Werte der wässrigen Formulierungen können zwischen 4 und 14, bevorzugt zwischen 5 und 11 und besonders bevorzugt zwischen 6 und 9 liegen. Wie bereits geschildert, bestimmt dabei der pH-Wert in der Formulierung die Funktion der LDH-Fixierungsschicht: bei pH < 6 tritt durch das langsame Auflösen des LDH's eine controlled-release Funktion zu Tage. Herrscht in der Formulierung hingegen ein pH > 6, dann ist das LDH stabil und dient so als fester "Behälter" für die gebundenen Anionen. Der Einsatzbereich bzw. die controlled-release Funktion wird natürlich durch eine bereits erwähnte, optional aufbringbare Nachbeschichtung maßgeblich beeinflusst. So kann eine Stabilisierung in einem breiteren pH-Werts-Bereich durch eine Nachbeschichtung einhergehen mit dem partiellen oder vollständigen Verlust der controlled-release-Funktion. Letzteres wird z. B. bevorzugt bei der Fixierung von UV-Filtern verwirklicht.
Organische UV-Filter werden in der Regel in einer Menge von 0.5 bis 10 Gewichtsprozent, vorzugsweise 1-8 %, anorganische Filter von 0.1 bis 30% in kosmetische Formulierungen eingearbeitet.
Den Konzentrationen der erfindungsgemäßen Hybridpigmente in der Formulierung sind keine Grenzen gesetzt. Sie können-je nach Anwendungsfall - zwischen 0.001 (rinse-off-Produkte, z.B. Duschgele) - 99% (z.B. Glanzeffekt-Artikel für besondere Anwendungen) liegen. Die erfindungsgemäßen Hybridpigmente können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erythrolose u.a.) sowie weitere kosmetische Wirkstoffe wie z.B. Bisabolol, LPO, Ectoin, Emblica, Allantoin, Bioflavanoide und deren Derivate. Generell können die erfindungsgemäßen Partikel auch mit den Stoffen-/Stoffgemischen kombiniert werden, welche über die anionbindende Schicht auch auf dem Substrat gebunden sind. Hierdurch werden z. B. Formulierungen verfügbar, bei der eine unmittelbare Wirkung nach Applizierung der Formulierung einhergeht (durch nicht gebundene Wirkstoffe), mit einer zusätzlichen verzögerten Wirkung (controlled release von LDH-gebundenen Wirkstoffen) über einen längeren Zeitraum.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Up, Pflegecremes und Sonnenschutzpräparaten eingesetzt werden.

Als Anwendungsform der kosmetischen Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie z.B. Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Die kosmetischen Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Weitere Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Feste Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Bei Einsatz der Partikel in Lacken und Farben sind alle dem Fachmann bekannten Anwendungsbereiche möglich, wie z.B. Pulverlacke, Automobillacke, Druckfarben für den Tief-, Offset-, Sieb- oder Flexodruck sowie für Lacke in Außenanwendungen. Die Lacke und Farben können hierbei beispielsweise strahlungshärtend, physikalisch trocknend oder chemisch härtend sein. Für die Herstellung der Druckfarben oder Flüssiglacke ist eine Vielzahl von Bindern, z.B. auf der Basis von Acrylaten, Methacrylaten, Polyestern, Polyurethanen, Nitrocellulose, Ethylcellulose, Polyamid, Polyvinylbutyrat, Phenolharzen, Maleinharzen, Stärke oder Polyvinylalkohol, Aminharzen, Alkydharzen, Epoxidharzen, Polytetrafluorethylen, Polyvinylidenfluoriden, Polyvinylchlorid oder Mischungen hieraus geeignet, insbesondere wasserlösliche Typen. Bei den Lacken kann es sich um Pulverlacke oder wasser- oder lösemittelbasierte Lacke handeln, wobei die Auswahl der Lackbestandteile dem Allgemeinwissen des Fachmanns unterliegt. Gängige polymere Bindemittel für Pulverlacke sind beispielsweise Polyester, Epoxide, Polyurethane, Acrylate oder Mischungen hieraus.

Darüber hinaus können die erfindungsgemäßen Partikel in Folien und Kunststoffen verwendet werden, so z.B. in Agrarfolien, infrarotreflektierenden Folien und Scheiben, Geschenkfolien, Kunststoffbehältnissen und Formkörpern für alle dem Fachmann bekannten Anwendungen. Als Kunststoffe eignen sich alle gängigen Kunststoffe für die Einarbeitung der erfindungsgemäßen Partikel, z.B. Duromere oder thermoplastische Kunststoffe. Die Beschreibung der Anwendungsmöglichkeiten und der einsetzbaren Kunststoffe, Verarbeitungsverfahren und Additive finden sich z.B. in der RD 472005 oder in R. Glausch, M. Kieser, R. Maisch, G. Pfaff, J. Weitzel, Perlglanzpigmente, Curt R. Vincentz Verlag, 1996, 83 ff., deren Offenbarungsgehalt hier mit umfasst ist.

Darüber hinaus eignen sich die erfindungsgemäßen Partikel auch für den Einsatz im Sicherheitsdruck und in sicherheitsrelevanten Merkmalen für z.B. fälschungssichere Karten und Ausweise, wie z.B. Eintrittskarten, Personalausweise, Geldscheine, Schecks und Scheckkarten sowie für andere fälschungssichere Dokumente. Im Bereich der Landwirtschaft können die Partikel zur Einfärbung von Saatgut und anderen Ausgangsgütern verwendet werden, darüber hinaus im Lebensmittelbereich zur Pigmentierung von Lebensmitteln. Zur Pigmentierung von Überzügen in Arzneimitteln wie z.B. Tabletten oder Dragees sind die erfindungsgemäßen Partikel ebenfalls einsetzbar.

Die erfindungsgemäßen Partikel eignen sich in den oben genannten Anwendungsgebieten ebenso zur Verwendung in Abmischungen mit organischen Farbstoffen und/oder Pigmenten, wie z.B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers) und herkömmlichen transparenten, bunten und schwarzen Glanzpigmenten auf der Basis von metalloxidbeschichteten Plättchen auf Basis von Glimmer, Glas, Al₂O₃, Fe₂O₃, SiO₂, etc. Beispiele und Ausführungsformen der oben genannten Materialien und Pigmentaufbauten finden sich z.B. auch in den Research Disclosures RD 471001 und RD 472005, deren Offenbarungen hiermit unter Bezugnahme mit eingeschlossen sind. Die erfindungsgemäßen Partikel können in jedem Verhältnis mit handelsüblichen Pigmenten und Füllern gemischt werden.

Als Füllstoffe sind z.B. natürlicher und synthetischer Glimmer, Nylon Powder, reine oder gefüllte Melaninharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe zu nennen. Bezüglich der Partikelform des Füllstoffes gibt es keine Einschränkungen. Sie kann den Anforderungen gemäß z.B. plättchenförmig, sphärisch oder nadelförmig sein.

Die erfindungsgemäßen Partikel sind weiterhin geeignet zur Herstellung von fließfähigen Pigmentpräparationen und Trockenpräparaten enthaltend ein oder mehrere erfindungsgemäße Partikel, Bindemittel und optional ein oder mehrere Additive. Unter Trockenpräparate sind auch Präparate zu verstehen, die 0 bis 8 Gew.-%, vorzugsweise 2 bis 8 Gew.-%, insbesondere 3 bis 6 Gew.-%, an Wasser und/oder eines Lösemittels oder Lösemittelgemisches enthalten. Die Trockenpräparate liegen vorzugsweise als Pellets, Granulate, Chips, Würstchen oder Briketts vor und weisen Teilchengrößen von 0.2-80 mm auf. Die Trockenpräparate finden insbesondere Anwendung bei der Herstellung von Druckfarben und in kosmetischen Formulierungen.

Kosmetika, Pharmazeutika, Formulierungen, Lacke, Farben, Kunststoffe, Folien, Dokumente und Ausweise, Saatgut, Lebensmittel oder Arzneimittelüberzüge sowie Pigmentpräparationen und Trockenpräparate enthaltend Partikel gemäß der vorliegenden Erfindung sind ein weiterer Gegenstand der vorliegenden Erfindung.

Die vollständige Offenbarung aller vorstehend genannten Patentanmeldungen, Patente und Veröffentlichungen ist durch Bezugnahme in dieser Anmeldung enthalten.

Die nachfolgenden Beispiele sollen die Erfindung näher erläutern, ohne sie jedoch zu begrenzen.

### Beispiele:

### Beispiel 1:

50 g Glasplättchen (ECR-Glas; Fraktion: 10 - 100 µm, Substrat-Dicke: 870 nm) werden zu 300 ml Wasser und 150 ml NaOH (0.5 M) zugegeben. Nach 30 min Rühren werden 2.9 g C.I. FD&C Red 6 "Unipure Red LC303" als Farbstoff und 2.9 g Neo Heliopan AP als UV-A-Filter zugegeben. 10.15 g MgCl₂ x 6H₂O, 6.05 g AlCl₃ x 6 H₂O und 20.95 g Harnstoff werden in je ca. 100 ml Wasser gelöst und ebenfalls hinzugegeben. Es wird für 24 h unter Rückfluss gerührt. Man lässt die Suspension abkühlen, saugt den Rückstand ab und wäscht mit Wasser. Der Rückstand wird bei 50°C getrocknet.

Man erhält glitzernde Partikel von brillant-roter Farbe, welche durch den Gehalt an organischem UV-Filter eine zusätzliche Absorptionsbande im UV-A-Bereich aufweisen.

### Beispiel 2:

50 g eines roten Interferenzpigmentes (ECR-Glas beschichtet mit ca. 4 Gew.-% SiO₂, ca. 1 Gew.-% SnO₂ und ca. 25 Gew.-% TiO₂; Fraktion: 10-100 µm, Substrat-Dicke: 850 nm) werden zu 190 ml Wasser und 310 ml NaOH (0.5 M) zugegeben. Nach 30 min Rühren werden 4 g Rutinsulfat (ein spezifisches Bioflavonoid) als Wirkstoff zugegeben. 10.15 g MgCl₂ x 6H₂0 werden in ca. 150 ml Wasser gelöst, mit 9.3 g FeCl₃-Lösung mit 15 Gew.-% Fe gemischt und innerhalb einer Stunde unter Rühren zugegeben. Anschließend wird mit Wasser auf ein Gesamtvolumen von 750 ml aufgefüllt. Es wird für 12 h unter Rückfluss gerührt. Man lässt die Suspension abkühlen, saugt den Rückstand ab und wäscht mit Wasser bis das Filtrat nahezu Wirkstofffrei abläuft. Der Rückstand wird bei 60°C getrocknet.

Man erhält ein glitzerndes Pigment mit roter Interferenzfarbe, welches bei einem pH-Wert < 6 in der Formulierung die antioxidative Wirkung von Rutinsulfat freisetzt.

### Beispiel 3:

a) 50 g Timiron® Splendid Red (ein mehrschichtiges rotes Interferenzpigment auf Basis von Glimmer der Fraktion 10-60 µm) werden zu 190 ml Wasser und 310 ml NaOH (0.5 M) zugegeben und 30 min gerührt. 7.34 g CaCl₂ x 2H₂0 und 6,05 g AlCl₃ x 6H₂0 werden in ca. 200 ml Wasser gelöst und innerhalb einer Stunde unter Rühren zugegeben. Anschließend wird mit Wasser auf ein Gesamtvolumen von 750 ml aufgefüllt. Es wird für 12 h unter Rückfluss gerührt. Man lässt die Suspension abkühlen, saugt den Rückstand ab und wäscht mit ca. zwei Liter Wasser. Der Rückstand wird zunächst bei 110°C getrocknet und anschließend bei 300- 600°C kalziniert.
b) Bei pH = 7,5 - 8,0 werden 2.9 g Phenylbenzimidazoliumsulfonsäure (Eusolex^{®} 232, ein UV-B-Filter) in 500 ml Wasser gelöst. Nun werden 25 g Zwischenprodukt aus Schritt a) zugegeben. Die Suspension wird 12 Stunden gerührt, abgesaugt, mit Wasser gewaschen bis im Filtrat kein UV-Filter mehr nachweisbar ist und das Produkt anschließend bei 50°C getrocknet.

Man erhält ein Pigment mit schwach roter Interferenzfarbe, welches durch den Gehalt an UV-Filter eine zusätzliche Absorptionsbande im UV-B-Bereich aufweist.

## Patentansprüche

1. Partikel, **dadurch gekennzeichnet, dass** sie Substrate und eine anionbindende Schicht umfassen, wobei die anionbindende Schicht ein oder mehrere anionbildende organische Wirkstoffe enthält und der organische Wirkstoff ausgewählt ist aus der Gruppe der Arzneiwirkstoffe, der Nahrungsergänzungsmittel, der diagnostischen Stoffe und Verbindungen und/oder der kosmetischen Wirkstoffe.

2. Partikel gemäß Anspruch 1, **dadurch gekennzeichnet, dass** die Substrate mit der anionbindenden Schicht beschichtet sind.

3. Partikel gemäß Anspruch 1 oder 2, **dadurch gekennzeichnet, dass** die anionbindende Schicht ein schichtförmiges Doppelhydroxid umfasst.

4. Partikeln gemäß einem oder mehreren der Ansprüche 1 bis 3, **dadurch gekennzeichnet, dass** das Substrat plättchenförmig, sphärisch, nadelförmig oder unregelmäßig geformt ist.

5. Partikel gemäß einem oder mehreren der Ansprüche 1 bis 4, **dadurch gekennzeichnet, dass** der organische Wirkstoff eine oder mehrere anionbildende Phenolat-, Enolat-, Carboxylat-, Sulfat-, Sulfonat-, Sulfinat-, Dithiolat-, Phosphat- und/oder Phosphit-Struktureinheiten enthält.

6. Partikel gemäß einem oder mehreren der Ansprüche 1 bis 5, **dadurch gekennzeichnet, dass** der organische Wirkstoff mindestens zwei anionbildende Phenolat, Enolat-, Carboxylat-, Sulfat-, Sulfonat-, Sulfinat-, Dithiolat-, Phosphat- und/oder Phosphit-Struktureinheiten enthält.

7. Partikel gemäß einem oder mehreren der Ansprüche 1 bis 6, **dadurch gekennzeichnet, dass** auf den Substraten und unterhalb der anionbindenden Schicht eine oder mehrere transparente, semitransparente und/oder opake Schichten aus Metalloxiden, Metalloxidhydraten, Metallsuboxiden, Metallen, Metallfluoriden, Metallnitriden, Metalloxynitriden oder Mischungen dieser Materialien aufgebracht sind.

8. Partikel gemäß einem oder mehreren der Ansprüche 1 bis 7, **dadurch gekennzeichnet, dass** die Substrate mit Ionen oder Elementen dotiert sind.

9. Partikel gemäß einem oder mehreren der Ansprüche 1 bis 8, **dadurch gekennzeichnet, dass** die anionbindende Schicht ein Doppelhydroxid der allgemeinen Formel
M²⁺₁₋ₓM³⁺ₓ(OH)₂(Zⁿ⁻)_{x/n} · mH₂O
umfasst, mit 0,2 < x < 0,33, wobei
M³⁺ ausgewählt ist aus Al³⁺, Cr³⁺, Fe³⁺, Ga³⁺, In³⁺, Y³⁺, La³⁺ und/oder Ce³⁺ und
M²⁺ ausgewählt ist aus Ba²⁺, Ca²⁺, Cu²⁺, Mg²⁺, Sr²⁺ und/oder Zn²⁺,
Z ein Gegenion der Metall-Salze und/oder ein Anion- oder ein Anionengemisch der anionbildenden organischen Wirkstoffe bedeutet, mit n für die Ladungszahl des Anions.

10. Partikel gemäß Anspruch 9, **dadurch gekennzeichnet, dass** M³⁺ = Al³⁺, Fe³⁺ ist und M²⁺ = Mg²⁺, Ca²⁺ oder Zn²⁺ ist.

11. Partikel gemäß einem oder mehreren der Ansprüche 1 bis 10, **dadurch gekennzeichnet, dass** die Schichtdicke der anionbindenden Schicht 0,5 - 500 nm beträgt.

12. Partikel gemäß einem oder mehreren der Ansprüche 1 bis 11, **dadurch gekennzeichnet, dass** der Anteil des anionbildenden Wirkstoffes oder Wirkstoffgemisches 0,001 bis 50 Gew.-%, insbesondere 0,5 - 20 Gew.-%, bezogen auf die Gesamtpartikel, beträgt.

13. Partikeln gemäß einem oder mehreren der Ansprüche 1 bis 12, **dadurch gekennzeichnet, dass** die anionbindende Schicht neben dem Wirkstoff zusätzlich anion-bildende organische, anorganische und/oder metallorganische Farbmittel enthält.

14. Partikel gemäß einem oder mehreren der Ansprüche 1 bis 13, **dadurch gekennzeichnet, dass** der Anteil der anionbildenden organischen, anorganischen und/oder metallorganischen Farbmittel 0,01 bis 30 Gew.-%, insbesondere 0,5-10 Gew.-%, bezogen auf die Gesamtpartikel, beträgt.

15. Partikel gemäß einem der Ansprüche 1 bis 14, **dadurch gekennzeichnet, dass** auf den Partikeln zusätzlich eine stabilisierende anorganische und/oder organische Beschichtung aufgebracht ist.

16. Verfahren zur Herstellung von Partikeln gemäß einem oder mehreren der Ansprüche 1 bis 15, wobei eine Suspension von Substraten, Metallkation-Salzen, Wirkstoffsalz(en) und gegebenenfalls Farbmittelsalz(en) sowie Laugen und/oder Harnstoff in einem Lösungsmittel oder Lösungsmittelgemisch bei einer Temperatur von 50 -120°C gerührt wird, so dass sich auf dem Substrat die anion-bindende Schicht bildet, wobei die anionbindende Schicht anion-bildende organische Wirkstoffe, und gegebenenfalls Farbmittel, enthält, anschließend das Produkt abgetrennt, gewaschen, getrocknet und gegebenenfalls gesiebt wird.

17. Verfahren nach Anspruch 16, **dadurch gekennzeichnet, dass** eine Lösung der Metallkation-Salze zu einer Suspension der Substrate, Wirkstoffsalze und gegebenenfalls Farbmittelsalz(en) sowie Laugen und/oder Harnstoff zugegeben wird.

18. Verfahren zur Herstellung von Partikeln gemäß einem oder mehreren der Ansprüche 1 bis 15, wobei eine Suspension von Substraten, Metallkation-Salzen sowie Laugen und/oder Harnstoff in einem Lösungsmittel oder Lösungsmittelgemisch bei einer Temperatur von 50 - 120°C gerührt wird, so dass sich auf dem Substrat die anionbindende Schicht bildet, danach das Produkt abgetrennt, gewaschen, getrocknet und gegebenenfalls gesiebt wird und dieses Produkt anschließend zu einer Lösung der anionbildenden organischen Wirkstoffe und gegebenenfalls anionbildenden Farbmittel unter Rühren hinzu gegeben wird.

19. Verfahren gemäß Anspruch 18, **dadurch gekennzeichnet, dass** das Produkt mit der auf dem Substrat gebildeten anionbindenden Schicht vor Zugabe zu der Lösung der anionbildenden organischen Wirkstoffe und gegebenenfalls anionbildenden Farbmittel bei Temperaturen von 300-600°C kalziniert wird.

20. Verwendung von Partikeln gemäß einem oder mehreren der Ansprüche 1 bis 15 in Kosmetika, Pharmazeutika, Formulierungen, Lacken, Farben, Kunststoffen, Folien, im Sicherheitsdruck, in Sicherheitsmerkmalen in Dokumenten und Ausweisen, zur Saatguteinfärbung, zur Lebensmitteleinfärbung oder in Arzneimittelüberzügen sowie zur Herstellung von Pigmentpräparationen und Trockenpräparaten.

21. Kosmetika, Pharmazeutika, Formulierungen, Lacke, Farben, Kunststoffe, Folien, Dokumente und Ausweise, Saatgut, Lebensmittel oder Arzneimittelüberzüge sowie Pigmentpräparationen und Trockenpräparate enthaltend Partikel gemäß einem oder mehreren der Ansprüche 1 bis 15.

## Claims

1. Particles, **characterised in that** they comprise substrates and an anion-binding layer, where the anion-binding layer comprises one or more anion-forming organic active compounds and the organic active compound is selected from the group of the medicament active compounds, the food supplements, the diagnostic substances and compounds and/or the cosmetic active compounds.

2. Particles according to Claim 1, **characterised in that** the substrates have been coated with the anion-binding layer.

3. Particles according to Claim 1 or 2, **characterised in that** the anion-binding layer comprises a layered double hydroxide.

4. Particles according to one or more of Claims 1 to 3, **characterised in that** the substrate is flake-form, spherical, needle-shaped or irregularly shaped.

5. Particles according to one or more of Claims 1 to 4, **characterised in that** the organic active compound contains one or more anion-forming phenolate, enolate, carboxylate, sulfate, sulfonate, sulfinate, dithiolate, phosphate and/or phosphite structural units.

6. Particles according to one or more of Claims 1 to 5, **characterised in that** the organic active compound contains at least two anion-forming phenolate, enolate, carboxylate, sulfate, sulfonate, sulfinate, dithiolate, phosphate and/or phosphite structural units.

7. Particles according to one or more of Claims 1 to 6, **characterised in that** one or more transparent, semitransparent and/or opaque layers of metal oxides, metal oxide hydrates, metal suboxides, metals, metal fluorides, metal nitrides, metal oxynitrides or mixtures of these materials have been applied to the substrates and below the anion-binding layer.

8. Particles according to one or more of Claims 1 to 7, **characterised in that** the substrates have been doped with ions or elements.

9. Particles according to one or more of Claims 1 to 8, **characterised in that** the anion-binding layer comprises a double hydroxide of the general formula
M²⁺₁₋ₓM³⁺ₓ(OH)₂(Zⁿ⁻)_{x/n} · mH₂O
where 0.2 < x < 0.33, where
M³⁺ is selected from Al³⁺, Cr³⁺, Fe³⁺, Ga³⁺, In³⁺, Y³⁺, La³⁺ and/or Ce³⁺ and
M²⁺ is selected from Ba²⁺, Ca²⁺, Cu²⁺, Mg²⁺, Sr²⁺ and/or Zn²⁺,
Z denotes a counterion of the metal salts and/or an anion or anion mixture of the anion-forming organic active compounds, where n stands for the charge number of the anion.

10. Particles according to Claim 9, **characterised in that** M³⁺ = Al³⁺, Fe³⁺ and M²⁺ = Mg²⁺, Ca²⁺ or Zn²⁺.

11. Particles according to one or more of Claims 1 to 10, **characterised in that** the layer thickness of the anion-binding layer is 0.5 - 500 nm.

12. Particles according to one or more of Claims 1 to 11, **characterised in that** the proportion of the anion-forming active compound or active-compound mixture is 0.001 to 50% by weight, in particular 0.5 - 20% by weight, based on the particles as a whole.

13. Particles according to one or more of Claims 1 to 12, **characterised in that** the anion-binding layer, besides the active compound, additionally comprises anion-forming organic, inorganic and/or organometallic colorants.

14. Particles according to one or more of Claims 1 to 13, **characterised in that** the proportion of the anion-forming organic, inorganic and/or organometallic colorants is 0.01 to 30% by weight, in particular 0.5-10% by weight, based on the particles as a whole.

15. Particles according to one of Claims 1 to 14, **characterised in that** a stabilising inorganic and/or organic coating has additionally been applied to the particles.

16. Process for the production of particles according to one or more of Claims 1 to 15, in which a suspension of substrates, metal cation salts, active-compound salt(s) and optionally colorant salt(s) and lyes and/or urea in a solvent or solvent mixture is stirred at a temperature of 50-120°C so that the anion-binding layer forms on the substrate, where the anion-binding layer comprises anion-forming organic active compounds and optionally colorants, and the product is subsequently separated off, washed, dried and optionally sieved.

17. Process according to Claim 16, **characterised in that** a solution of the metal cation salts is added to a suspension of the substrates, active-compound salts and optionally colorant salt(s) and lyes and/or urea.

18. Process for the production of particles according to one or more of Claims 1 to 15, in which a suspension of substrates, metal cation salts and lyes and/or urea in a solvent or solvent mixture is stirred at a temperature of 50-120°C so that the anion-binding layer forms on the substrate, the product is then separated off, washed, dried and optionally sieved, and this product is subsequently added to a solution of the anion-forming organic active compounds and optionally anion-forming colorants with stirring.

19. Process according to Claim 18, **characterised in that** the product is calcined at temperatures of 300-600°C with the anion-binding layer formed on the substrate before addition to the solution of the anion-forming organic active compounds and optionally anion-forming colorants.

20. Use of particles according to one or more of Claims 1 to 15 in cosmetics, pharmaceuticals, formulations, paints, coatings, plastics, films, in security printing, in security features in documents and identity papers, for colouring seed, for colouring foods or in medicament coatings and for the preparation of pigment compositions and dry preparations.

21. Cosmetics, pharmaceuticals, formulations, paints, coatings, plastics, films, documents and identity papers, seed, foods or medicament coatings and pigment compositions and dry preparations comprising particles according to one or more of Claims 1 to 15.

## Revendications

1. Particules, **caractérisées en ce qu'**elles comprennent des substrats et une couche fixatrice d'anions, où la couche fixatrice d'anions comprend un ou plusieurs composés actifs organiques formateurs d'anions et le composé actif organique est choisi dans le groupe constitué par les composés actifs médicamenteux, les compléments alimentaires, les substances et composés de diagnostic et/ou les composés actifs cosmétiques.

2. Particules selon la revendication 1, **caractérisées en ce que** les substrats ont été revêtus de la couche fixatrice d'anions.

3. Particules selon la revendication 1 ou 2, **caractérisées en ce que** la couche fixatrice d'anions comprend un hydroxyde double lamellaire.

4. Particules selon l'une ou plusieurs parmi les revendications 1 à 3, **caractérisées en ce que** le substrat est sous forme de paillettes, sphérique, sous forme d'aiguilles ou de forme irrégulière.

5. Particules selon l'une ou plusieurs parmi les revendications 1 à 4, **caractérisées en ce que** le composé actif organique contient un ou plusieurs motifs structurels phénolate, énolate, carboxylate, sulfate, sulfonate, sulfinate, dithiolate, phosphate et/ou phosphite formateurs d'anions.

6. Particules selon l'une ou plusieurs parmi les revendications 1 à 5, **caractérisées en ce que** le composé actif organique contient au moins deux motifs structurels phénolate, énolate, carboxylate, sulfate, sulfonate, sulfinate, dithiolate, phosphate et/ou phosphite formateurs d'anions.

7. Particules selon l'une ou plusieurs parmi les revendications 1 à 6, **caractérisées en ce qu'**une ou plusieurs couches transparentes, semi-transparentes et/ou opaques d'oxydes métalliques, d'hydrates d'oxydes métalliques, de suboxydes métalliques, de métaux, de fluorures métalliques, de nitrures métalliques, d'oxynitrures métalliques, ou de mélanges de ces matériaux, ont été appliquées aux substrats et sous la couche fixatrice d'anions.

8. Particules selon l'une ou plusieurs parmi les revendications 1 à 7, **caractérisées en ce que** les substrats ont été dopés par des ions ou des éléments.

9. Particules selon l'une ou plusieurs parmi les revendications 1 à 8, **caractérisées en ce que** la couche fixatrice d'anions comprend un hydroxyde double de formule générale
M²⁺₁₋ₓM³⁺ₓ(OH)₂(Zⁿ⁻)_{x/n} · mH₂O
dans laquelle 0,2 < x < 0,33, où
M³⁺ est choisi parmi Al³⁺, Cr³⁺, Fe³⁺, Ga³⁺, In³⁺, Y³⁺, La³⁺ et/ou Ce³⁺ and
M²⁺ est choisi parmi Ba²⁺, Ca²⁺, Cu²⁺, Mg²⁺, Sr²⁺ et/ou Zn²⁺,
Z désigne un contre-ion des sels métalliques et/ou un anion ou un mélange d'anions des composés actifs organiques formateurs d'anions, où n représente le nombre de charge de l'anion.

10. Particules selon la revendication 9, **caractérisées en ce que** M³⁺ = Al³⁺, Fe³⁺ et M²⁺ = Mg²⁺, Ca²⁺ ou Zn²⁺.

11. Particules selon l'une ou plusieurs parmi les revendications 1 à 10, **caractérisées en ce que** l'épaisseur de couche de la couche fixatrice d'anions est de 0,5 - 500 nm.

12. Particules selon l'une ou plusieurs parmi les revendications 1 à 11, **caractérisées en ce que** la proportion du composé actif ou mélange de composés actifs formateur d'anion va de 0,001 à 50% en poids, en particulier est de 0,5 - 20% en poids, sur la base de l'ensemble des particules.

13. Particules selon l'une ou plusieurs parmi les revendications 1 à 12, **caractérisées en ce que** la couche fixatrice d'anions, outre le composé actif, comprend en outre des colorants organiques, inorganiques et/ou organométalliques formateurs d'anions.

14. Particules selon l'une ou plusieurs parmi les revendications 1 à 13, **caractérisées en ce que** la proportion des colorants organiques, inorganiques et/ou organométalliques formateurs d'anions va de 0,01 à 30% en poids, en particulier est de 0.5-10% en poids, sur la base de l'ensemble des particules.

15. Particules selon l'une des revendications 1 à 14, **caractérisées en ce qu'**un revêtement inorganique et/ou organique stabilisant a été de plus appliqué aux particules.

16. Procédé de production de particules selon l'une ou plusieurs parmi les revendications 1 à 15, dans lequel une suspension de substrats, de sels de cations métalliques, d'un ou plusieurs sels de composés actifs et éventuellement d'un ou plusieurs sels de colorants et de lessives et/ou d'urée dans un solvant ou un mélange de solvants est agitée à une température de 50-120°C de sorte que la couche fixatrice d'anions se forme sur le substrat, où la couche fixatrice d'anions comprend des composés actifs organiques formateurs d'anions et éventuellement des colorants, et le produit est ensuite séparé, lavé, séché et éventuellement tamisé.

17. Procédé selon la revendication 16, **caractérisé en ce qu'**une solution des sels de cations métalliques est ajoutée à une suspension des substrats, des sels de composés actifs et éventuellement du ou des sels de colorants et de lessives et/ou d'urée.

18. Procédé de production de particules selon l'une ou plusieurs parmi les revendications 1 à 15, dans lequel une suspension de substrats, de sels de cations métalliques et de lessives et/ou d'urée dans un solvant ou un mélange de solvants est agitée à une température de 50-120°C de sorte que la couche fixatrice d'anions se forme sur le substrat, le produit est ensuite séparé, lavé, séché et éventuellement tamisé, et ce produit est ensuite ajouté à une solution des composés actifs organiques formateurs d'anions et éventuellement de colorants formateurs d'anions sous agitation.

19. Procédé selon la revendication 18, **caractérisé en ce que** le produit est calciné à des températures de 300-600°C, la couche fixatrice d'anions étant formée sur le substrat avant addition à la solution des composés actifs organiques formateurs d'anions et éventuellement de colorants formateurs d'anions.

20. Utilisation des particules selon l'une ou plusieurs parmi les revendications 1 à 15, dans les produits cosmétiques, les substances pharmaceutiques, les formulations, les peintures, les revêtements, les matières plastiques, les films, dans l'impression de sécurité, dans les dispositifs de sécurité dans des documents et des pièces d'identité, pour la coloration de semences, pour la coloration d'aliments ou dans des enrobages de médicaments et pour la préparation de compositions de pigments et de préparations sèches.

21. Produits cosmétiques, substances pharmaceutiques, formulations, peintures, revêtements, matières plastiques, films, documents et pièces d'identité, semences, aliments ou enrobages de médicaments, et compositions de pigments et préparations sèches comprenant des particules selon l'une ou plusieurs parmi les revendications 1 à 15.
